# EUROPEAN PATENT SPECIFICATION

(11) **EP 4 144 850 B1**
(45) Date of publication and mention of the grant of the patent: **25.11.2026**
(21) Application number: 22181154.0
(22) Date of filing: 23.12.2015
(51) Int. Cl.: C12N 15/82, A01H 1/08, A01H 5/06, A01H 5/10, A01H 6/20, C07K 14/415, C12N 15/29

(54) **GENERATION OF HAPLOID PLANTS**
ERZEUGUNG VON HAPLOIDEN PFLANZEN
GÉNÉRATION DE PLANTES HAPLOÏDES

(30) Priority: 23.12.2014 EP 14004389
(43) Date of publication of application: 08.03.2023
(60) Divisional application: 26157346.3 / 4 728 855
(62) Document number(s) of the earlier application(s) in accordance with Art. 76 EPC: 15817864.0 / 3 237 623
(73) Proprietor: KWS SAAT SE & Co. KGaA, 37574 Einbeck (DE)
(72) Inventor: BOLDUAN, Christof, 37574 Einbeck (DE); BREUER, Frank, 37574 Einbeck (DE); KLOIBER-MAITZ, Monika, 37574 Einbeck (DE); NIESSEN, Markus, 30880 Laatzen (DE); OUZUNOVA, Milena, 37077 Göttingen (DE); SCHULZ, Britta, 37574 Einbeck (DE); WIECKHORST, Silke, 37574 Einbeck (DE)
(74) Representative: Eisenführ Speiser

(56) References cited:
- WO-A1-2011/044132
- WO-A2-2014/110274
- MARUTHACHALAM RAVI & SIMON W L CHAN: "Haploid plants produced by centromere-mediated genome elimination", NATURE, NATURE PUBLISHING GROUP, UNITED KINGDOM, vol. 464, no. 7288, 25 March 2010 (2010-03-25), pages 615 - 620, XP002677783, ISSN: 0028-0836, DOI: 10.1038/NATURE08842
- BRENDA MARIN-RODRIGUEZ: "Can Point Mutations in Kinetochore Proteins Create Haploid Plants in Arabidopsis thaliana?", UC DAVIS EXPLORATIONS, September 2014 (2014-09-01), pages 7 - 7, XP055191293, Retrieved from the Internet <URL:http://explorations.ucdavis.edu/docs/2014/2014explorations.pdf> [retrieved on 20150522]
- TAKAYOSHI ISHII ET AL: "Functional characterization of barley CENH3 variants", PLANT MOLECULAR CYTOGENETICS IN GENOMIC AND POSTGENOMIC ERA, September 2014 (2014-09-01), pages 51, XP055191331, Retrieved from the Internet <URL:http://real.mtak.hu/18302/1/abstract_book.pdf> [retrieved on 20150522]
- "Plant Centromere Biology", 8 April 2013 (2013-04-08), Oxford [u.a.], pages 67 - 82, XP055163252, ISBN: 978-1-119-94921-3, Retrieved from the Internet <URL:http://onlinelibrary.wiley.com/store/10.1002/9781118525715.ch6/asset/ch6.pdf?v=1&t=i55012yy&s=13933095b8df6c4f37caff5cd3f546a86de9d9f1> DOI: 10.1002/9781118525715.ch6
- M. RAVI ET AL: "The Rapidly Evolving Centromere-Specific Histone Has Stringent Functional Requirements in Arabidopsis thaliana", GENETICS, vol. 186, no. 2, 13 July 2010 (2010-07-13), pages 461 - 471, XP055142479, ISSN: 0016-6731, DOI: 10.1534/genetics.110.120337
- POOJA BHATNAGAR- MATHUR ET AL: "Engineering Centromeres for Haploidy Induction in Grain Legumes", ICRISAT ASIA REGIONAL PLANNING MEETING, PATANCHERU, INDIA, FEBRUARY 10-12, 2014, February 2014 (2014-02-01), Patancheru, India, XP055193834, Retrieved from the Internet <URL:http://ksiconnect.icrisat.org/wp-content/uploads/2014/02/6Pooja-DH-for-haploids.pdf> [retrieved on 20150605]
- IZABEL C. R. MORAES: "Structural requirements for CENH3 targeting to centromeric chromatin", PH. D. THESIS, February 2011 (2011-02-01), Halle (Saale), Germany, XP055191122, Retrieved from the Internet <URL:http://d-nb.info/1025136047/34> [retrieved on 20150522]
- MARUTHACHALAM RAVI ET AL: "A haploid genetics toolbox for Arabidopsis thaliana", NATURE COMMUNICATIONS, vol. 5, 31 October 2014 (2014-10-31), pages 5334, XP055191112, DOI: 10.1038/ncomms6334
- RAHELEH KARIMI-ASHTIYANI ET AL: "Point mutation impairs centromeric CENH3 loading and induces haploid plants", PROCEEDINGS OF THE NATIONAL ACADEMY OF SCIENCES, vol. 112, no. 36, 20 August 2015 (2015-08-20), pages 11211 - 11216, XP055233810, ISSN: 0027-8424, DOI: 10.1073/pnas.1504333112

## Description

The present invention relates to a polynucleotide which comprises a nucleotide sequence encoding a centromer histone H3 (CENH3) protein, wherein the polynucleotide comprises at least one mutation causing an alteration of the amino acid sequence of the CENH3 protein and said alteration confers the biological activity of a haploid inducer and is a substitution of a specified amino acid in the loop2. Further, the present invention provides methods of generating the plants of the present invention.

The invention is as defined in the claims. Any aspects, embodiments and examples of the present disclosure which do not fall under the scope of the appended claims do not form part of the invention and are merely provided for illustrative purposes.

The generation and use of haploids is one of the most powerful biotechnological means to improve cultivated plants. The advantage of haploids for breeders is that homozygosity can be achieved already in the first generation after dihaploidization, creating doubled haploid plants, without the need of several backcrossing generations required to obtain a high degree of homozygosity. Further, the value of haploids in plant research and breeding lies in the fact that the founder cells of doubled haploids are products of meiosis, so that resultant populations constitute pools of diverse recombinant and at the same time genetically fixed individuals. The generation of doubled haploids thus provides not only perfectly useful genetic variability to select from with regard to crop improvement but is also a valuable means to produce mapping populations, recombinant inbreds as well as instantly homozygous mutants and transgenic lines.

Haploids can be obtained by *in vitro* or *in vivo* approaches. However, many species and genotypes are recalcitrant to these processes. Alternatively, substantial changes of the centromere-specific histone H3 variant (CENH3, also called CENP-A), by swapping its N-terminal regions and fusing it to GFP ("GFP-tailswap" CENH3), creates haploid inducer lines in the model plant *Arabidopsis thaliana* (Ravi and Chan, Nature, 464 (2010), 615-618; Comai, L, "Genome elimination: translating basic research into a future tool for plant breeding.", PLoS biology, 12.6 (2014)). CENH3 proteins are variants of H3 histone proteins that are members of the kinetochore complex of active centromeres. With these "GFP-tailswap" haploid inducer lines, haploidization occurred in the progeny when a haploid inducer plant was crossed with a wildtype plant. Interestingly, the haploid inducer line was stable upon selfing, suggesting that a competition between modified and wild type centromere in the developing hybrid embryo results in centromere inactivation of the inducer parent and consequently in uniparental chromosome elimination. As a result, the chromosomes containing the altered CENH3 protein are lost during early embryo development producing haploid progeny containing only the chromosomes of the wildtype parent.

Thus, haploid plants can be obtained by crossing "GFP-tailswap" transgenic plants as haploid inducer to wildtype plants. However, as described above, this technique requires substantial changes of the CENH3 protein and the plants comprise a heterologous transgene, which is economically problematic because of increasing public reluctance toward genetically engineered crops.

WO 2011/044132A1 and WO2014/110274A2 disclose methods and compositions for generating haploid plants by manipulating CENH3.

It is therefore an object of the present invention to overcome the aforementioned problems and in particular to provide alternative haploid inducer plants which do not comprise substantial modifications of their CENH3 protein and/or which are not genetically engineered.

This problem is solved by the subject matter of the independent claims. In the context of the present invention the term 'alteration' means any modification of the amino acid sequence of the protein CENH3 (including multiple modifications) which are caused by at least one mutation in the polynucleotide which comprises a nucleotide sequence encoding a centromer histone H3 (CENH3) protein. The polynucleotide can be a genomic DNA of the CENH3 gene, the cDNA of CENH3, or 5'- or 3'-untranslated regions of the CENH3 gene or a mixture thereof that comprises for instances a part of the genomic DNA and a part of the cDNA. An alteration can be a substitution of one or more amino acids, an insertion of one or more amino acids or a deletion of one or more amino acids. Mutations at DNA level which are able to alter the amino acid sequence of the protein CENH3 can be point mutations leading to an amino acid substitution or a stop codon, insertions or deletion which shift the reading frame of the CENH3 gene, or mutations in the splicing sites.

### Summary

Disclosed herein is a plant having biological activity of a haploid inducer and comprising a polynucleotide which comprises a nucleotide sequence encoding a centromer histone H3 (CENH3) protein, said polynucleotide comprises at least one mutation causing an alteration of the amino acid sequence of the CENH3 protein and said alteration confers the biological activity of a haploid inducer, having at least one mutation causing a substitution of a specified amino acid in the loop2, which can be defined as i.the amino acid arginine at position 159 of SEQ ID NO: 14 is substituted for histidine, or ii.the amino acid valine at position 160 of SEQ ID NO: 14 is substituted for isoleucine, or iii.the amino acid threonine at position 139 of SEQ ID NO: 20 is substituted for isoleucine. Crossing between the plant and a wildtype plant or a plant expressing wildtype CENH3 protein may yield at least 0.1 % haploid progeny. The polynucleotide comprising the at least one mutation may be an endogenous gene or a transgene. Further disclosed is a part of the plant, which is preferably a shoot vegetative organ, root, flower or floral organ, seed, fruit, ovule, embryo, plant tissue or cell.

In one aspect, the present invention relates to an isolated polynucleotide comprising a nucleotide sequence encoding a centromer histone H3 (CENH3) protein, and the polynucleotide comprises at least one mutation causing an alteration of the amino acid sequence of the CENH3 protein and said alteration confers the biological activity of a haploid inducer, and the at least one mutation causes a substitution of a specified amino acid in the loop2, which can be defined as i. the amino acid arginine at position 159 of SEQ ID NO: 14 is substituted for histidine, or ii. the amino acid valine at position 160 of SEQ ID NO: 14 is substituted for isoleucine, or iii. the amino acid threonine at position 139 of SEQ ID NO: 20 is substituted for isoleucine.

In another aspect, the present invention relates to a vector comprising said polynucleotide. In a preferred embodiment the present invention relates to a plant cell or host cell comprising the polynucleotide as defined herein as a transgene or the vector of as defined herein as a transgene.

Another aspect of the present invention concerns a method of generating a plant as defined herein, comprising the steps of: a) transforming a plant cell with the polynucleotide mentioned herein or the vector mentioned herein, and b) regenerating a plant having the biological activity of a haploid inducer from the plant cell.

In the context of the present invention, the at least one mutation causes the alteration of the amino acid sequence of the protein CENH3 which confers the biological activity of a haploid inducer in at least one segment of the amino acid sequence of the CENH3 protein. Such a sequence segment may be selected from the group consisting of N-terminal tail domain, CATD domain, aN-helix, α1-helix, loop1, α2-helix, loop2, α3-helix, and C-terminal domain, however, selected herein is loop2. The N-terminal tail domain of the CENH3 protein corresponds to amino acid sequence from position 1 to position 82 as set forth in SEQ ID NO: 11 derived from *Arabidopsis thaliana* and/or the N-terminal tail domain of the CENH3 protein is encoded by a nucleotide sequence corresponding to nucleotides from position 1 to position 246 as set forth in SEQ ID NO: 10 derived from *Arabidopsis thaliana.* The CATD domain of the CENH3 protein corresponds to an amino acid sequence from position 113 to position 155 as set forth in SEQ ID NO: 11 derived from *Arabidopsis thaliana* and/or the CATD domain of the CENH3 protein is encoded by a nucleotide sequence corresponding to nucleotides from position 337 to position 465 as set forth in SEQ ID NO: 10 derived from *Arabidopsis thaliana.* The aN-helix of the CENH3 protein corresponds to an amino acid sequence from position 83 to position 97 as set forth in SEQ ID NO: 11 derived from *Arabidopsis thaliana* and/or the aN-helix of the CENH3 protein is encoded by a nucleotide sequence corresponding to nucleotides from position 247 to position 291 as set forth in SEQ ID NO: 10 derived from *Arabidopsis thaliana.* The α1-helix of the CENH3 protein corresponds to an amino acid sequence from position 103 to position 113 as set forth in SEQ ID NO: 11 derived from *Arabidopsis thaliana* and/or the α1-helix of the CENH3 protein is encoded by a nucleotide sequence corresponding to nucleotides from position 307 to position 339 as set forth in SEQ ID NO: 10 derived from *Arabidopsis thaliana.* The loop1 of the CENH3 protein corresponds to an amino acid sequence from position 114 to position 126 as set forth in SEQ ID NO: 11 derived from *Arabidopsis thaliana* and/or the loop1 of the CENH3 protein is encoded by a nucleotide sequence corresponding to nucleotides from position 340 to position 378 as set forth in SEQ ID NO: 10 derived from *Arabidopsis thaliana.* The α2-helix of the CENH3 protein corresponds to an amino acid sequence from position 127 to position 155 as set forth in SEQ ID NO: 11 derived from *Arabidopsis thaliana* and/or the α2-helix of the CENH3 protein is encoded by a nucleotide sequence corresponding to nucleotides from position 379 to position 465 as set forth in SEQ ID NO: 10 derived from *Arabidopsis thaliana.* The loop2 of the CENH3 protein corresponds to an amino acid sequence from position 156 to position 162 as set forth in SEQ ID NO: 11 derived from *Arabidopsis thaliana* and/or the loop2 of the CENH3 protein is encoded by a nucleotide sequence corresponding to nucleotides from position 466 to position 486 as set forth in SEQ ID NO: 10 derived from *Arabidopsis thaliana.* The α3-helix of the CENH3 protein corresponds to an amino acid sequence from position 163 to position 172 as set forth in SEQ ID NO: 11 derived from *Arabidopsis thaliana* and/or the α3-helix of the CENH3 protein is encoded by a nucleotide sequence corresponding to nucleotides from position 487 to position 516 as set forth in SEQ ID NO: 10 derived from *Arabidopsis thaliana.* The C-terminal domain of the CENH3 protein corresponds to an amino acid sequence from position 173 to position 178 as set forth in SEQ ID NO: 11 derived from *Arabidopsis thaliana* and/or the C-terminal domain of the CENH3 protein is encoded by a nucleotide sequence corresponding to nucleotides from position 517 to position 534 as set forth in SEQ ID NO: 10 derived from *Arabidopsis thaliana.* The *A. thaliana* sequences serve only as references and do not limit the invention to the particular *A. thaliana* sequences. Due to the high level of conservation a person skilled in the art is able to find the nucleotide sequence and amino acid sequence corresponding to the *A. thaliana* sequences in any other plant material or plant species.

CENH3 proteins are variants of H3 histone proteins that are members of the kinetochore complex of active centromeres, i.e. the protein structure on chromosomes where spindle fibres attach during cell division. Basically, CENH3 proteins are characterized by a variable N-terminal tail domain, which does not form a rigid secondary structure, and a conserved histone fold domain consisting of three α-helical regions, termed α1 to α3, which are connected by two loop sections. The N-terminal tail domain is primarily subject to post translational modification by enzymes. Such modifications include methylation, citrullination, phosphorylation, SUMOylation, ubiquitination, and ADP-ribosylation and affect the function of regulation of the CENH3 gene. Within the histone fold domain, the highly conserved CATD domain (CENP-A targeting domain) is located, which is formed by parts of the α1-helix, the complete α2-helix and the connecting loop1. The conserved CATD domain is required for CENH3 loading by chaperones and thus vital for its kinetochore localization and centromere function. N-terminal tail domain and histone fold domain are linked by the aN-helix.

The present inventors surprisingly found that a plant possessing the capability to produce haploid progeny, i.e. a haploid inducer, can be obtained not only by alteration of the amino acid sequence of the conserved CENH3 protein, but also by alteration of the amino acid sequence of any other domain and structural regions of the CENH3 gene and CENH3 protein. In addition, the capability to produce haploid progeny can be further enhanced by combination of two or more alterations of the amino acid sequence of the CENH3 protein in different domains, segments or structural regions of the CENH3 protein. Hence, the efficiency of haploid production can be increased significantly. Advantageously, this can be achieved by transgenic as well as non-transgenic methods. Non-transgenic methods are preferred because of enormous **costs** for **deregulation** of genetically modified organisms (GMO) as well as increasing public rejection of genetically modified organisms (GMO) or plants generated by means of GMO, in particular crops for human consumption, and extensive market authorisation processes including rigorous safety assessments of such GMOs.

The disclosure provides a plant comprising and expressing a CENH3 protein, wherein the plant comprises a polynucleotide and the polynucleotide comprising the nucleotide sequence of the gene encoding the CENH3 protein comprises at least one mutation causing the alteration of the amino acid sequence of the CENH3 protein in at least one segment of the amino acid sequence of the CENH3 protein, wherein the segment is selected from the group consisting of the N-terminal tail domain (not claimed), most preferred the N-terminal tail domain having the consensus sequence of SEQ ID NO: 1 and SEQ ID NO: 2, the aN-helix (not claimed), most preferred the aN-helix having the consensus sequence of SEQ ID NO: 3, the α1-helix (not claimed), most preferred the α1-helix having the consensus sequence of SEQ ID NO: 4, the loop1 (not claimed), most preferred the loop1 having the consensus sequence of SEQ ID NO: 5, the α2-helix (not claimed), most preferred the α2-helix having the consensus sequence of SEQ ID NO: 6, the loop2 (selected herein), most preferred the loop2 having the consensus sequence of SEQ ID NO: 7, the α3-helix (not claimed), most preferred the α3-helix having the consensus sequence of SEQ ID NO: 8 and the C-terminal domain, most preferred the C-terminal domain having the consensus sequence of SEQ ID NO: 9. The alteration of the amino acid sequence of the CENH3 protein can confer the biological activity of a haploid inducer to the plant. The disclosure relates to a plant comprising a polynucleotide which comprises a nucleotide sequence encoding a centromer histone H3 (CENH3) protein, wherein the polynucleotide comprises at least one mutation and wherein the at least one mutation causes an alteration of the amino acid sequence of the CENH3 protein in at least one segment of the amino acid sequence of the CENH3 protein. The segment can be a) the N-terminal tail domain which is encoded by a nucleotide sequence corresponds to nucleotides from position 1 to position 246 as set forth in SEQ ID NO: 10 derived from *Arabidopsis* thaliana, corresponds to amino acid sequence from positions 1 to position 82 as set forth in SEQ ID NO: 11 derived from *Arabidopsis thaliana,* or is encoded by a nucleotide sequence which corresponds to nucleotides from position 1 to position 177 as set forth in SEQ ID NO: 22 derived from *Beta vulgaris,* corresponds to an amino acid sequence from positions 1 to position 59 as set forth in SEQ ID NO: 23 derived from *Beta vulgaris,* or is encoded by a nucleotide sequence which corresponds to nucleotides from position 1 to position 252 as set forth in SEQ ID NO: 13 derived from *Brassica napus,* which corresponds to an amino acid sequence from positions 1 to position 84 as set forth in SEQ ID NO: 14 derived from *Brassica napus,* or is encoded by a nucleotide sequence which corresponds to nucleotides from position 1 to position 186 as set forth in SEQ ID NO: 19 derived from *Zea mays,* corresponds to an amino acid sequence from positions 1 to position 62 as set forth in SEQ ID NO: 20 derived from *Zea mays,* or is encoded by a nucleotide sequence which corresponds to nucleotides from position 1 to position 186 as set forth in SEQ ID NO: 16 derived from *Sorghum bicolor,* corresponds to amino acid sequence from positions 1 to position 62 as set forth in SEQ ID NO: 17 derived from *Sorghum bicolor,* or having the consensus sequence of SEQ ID NO: 1 and SEQ ID NO: 2, b) the αN-helixwhich is encoded by a nucleotide sequence corresponds to nucleotides from position 247 to position 291 as set forth in SEQ ID NO: 10 derived from *Arabidopsis thaliana,* corresponds to an amino acid sequence from positions 83 to position 97 as set forth in SEQ ID NO: 11 derived from *Arabidopsis thaliana,* or is encoded by a nucleotide sequence which corresponds to nucleotides from position 178 to position 222 as set forth in SEQ ID NO: 22 derived from *Beta vulgaris,* corresponds to an amino acid sequence from positions 60 to position 74 as set forth in SEQ ID NO: 23 derived from *Beta vulgaris,* or is encoded by a nucleotide sequence which corresponds to nucleotides from position 253 to position 297 as set forth in SEQ ID NO: 13 derived from *Brassica napus,* corresponds to an amino acid sequence from positions 85 to position 99 as set forth in SEQ ID NO: 14 derived from *Brassica napus,* or is encoded by a nucleotide sequence which corresponds to nucleotides from position 187 to position 231 as set forth in SEQ ID NO: 19 derived from *Zea mays,* corresponds to an amino acid sequence from positions 63 to position 77 as set forth in SEQ ID NO: 20 derived from *Zea mays,* or is encoded by a nucleotide sequence which corresponds to nucleotides from position 187 to position 231 as set forth in SEQ ID NO: 16 derived from *Sorghum bicolor,* corresponds to an amino acid sequence from positions 63 to position 77 as set forth in SEQ ID NO: 17 derived from *Sorghum bicolor,* or having the consensus sequence of SEQ ID NO: 3, c) the α1-helix which is encoded by a nucleotide sequence corresponds to nucleotides from position 307 to position 339 as set forth in SEQ ID NO: 10 derived from *Arabidopsis thaliana,* corresponds to an amino acid sequence from positions 103 to position 113 as set forth in SEQ ID NO: 11 derived from *Arabidopsis thaliana,* or is encoded by a nucleotide sequence which corresponds to nucleotides from position 238 to position 270 as set forth in SEQ ID NO: 22 derived from *Beta vulgaris,* corresponds to an amino acid sequence from positions 80 to position 90 as set forth in SEQ ID NO: 23 derived from *Beta vulgaris,* or is encoded by a nucleotide sequence which corresponds to nucleotides from position 313 to position 345 as set forth in SEQ ID NO: 13 derived from *Brassica napus,* corresponds to an amino acid sequence from positions 105 to position 115 as set forth in SEQ ID NO: 14 derived from *Brassica napus,* or is encoded by a nucleotide sequence which corresponds to nucleotides from position 247 to position 279 as set forth in SEQ ID NO: 19 derived from *Zea mays,* corresponds to an amino acid sequence from positions 83 to position 93 as set forth in SEQ ID NO: 20 derived from *Zea mays,* or is encoded by a nucleotide sequence corresponds to nucleotides from position 247 to position 279 as set forth in SEQ ID NO: 16 derived from *Sorghum bicolor,* corresponds to an amino acid sequence from positions 83 to position 93 as set forth in SEQ ID NO: 17 derived from *Sorghum bicolor,* or having the consensus sequence of SEQ ID NO: 4, d) the loop1 which is encoded by a nucleotide sequence which corresponds to nucleotides from position 340 to position 378 as set forth in SEQ ID NO: 10 derived from *Arabidopsis thaliana,* corresponds to an amino acid sequence from positions 114 to position 126 as set forth in SEQ ID NO: 11 derived from *Arabidopsis thaliana,* or is encoded by a nucleotide sequence which corresponds to nucleotides from position 271 to position 306 as set forth in SEQ ID NO: 22 derived from *Beta vulgaris,* corresponds to an amino acid sequence from positions 91 to position 102 as set forth in SEQ ID NO: 23 derived from *Beta vulgaris,* or is encoded by a nucleotide sequence which corresponds to nucleotides from position 346 to position 384 as set forth in SEQ ID NO: 13 derived from *Brassica napus,* corresponds to an amino acid sequence from positions 116 to position 128 as set forth in SEQ ID NO: 14 derived from *Brassica napus,* or is encoded by a nucleotide sequence which corresponds to nucleotides from position 280 to position 318 as set forth in SEQ ID NO: 19 derived from *Zea mays,* corresponds to an amino acid sequence from positions 94 to position 106 as set forth in SEQ ID NO: 20 derived from *Zea mays,* or is encoded by a nucleotide sequence which corresponds to nucleotides from position 280 to position 318 as set forth in SEQ ID NO: 16 derived from *Sorghum bicolor,* corresponds to an amino acid sequence from positions 94 to position 106 as set forth in SEQ ID NO: 17 derived from *Sorghum bicolor,* or having the consensus sequence of SEQ ID NO: 5, e) the α2-helix which is encoded by a nucleotide sequence which corresponds to nucleotides from position 379 to position 465 as set forth in SEQ ID NO: 10 derived from *Arabidopsis* thaliana, corresponds to an amino acid sequence from positions 127 to position 155 as set forth in SEQ ID NO: 11 derived from *Arabidopsis thaliana,* or is encoded by a nucleotide sequence which corresponds to nucleotides from position 307 to position 393 as set forth in SEQ ID NO: 22 derived from *Beta vulgaris,* corresponds to an amino acid sequence from positions 103 to position 131 as set forth in SEQ ID NO: 23 derived from *Beta vulgaris,* or is encoded by a nucleotide sequence which corresponds to nucleotides from position 385 to position 471 as set forth in SEQ ID NO: 13 derived from *Brassica napus,* corresponds to an amino acid sequence from positions 129 to position 157 as set forth in SEQ ID NO: 14 derived from *Brassica napus,* or is encoded by a nucleotide sequence which corresponds to nucleotides from position 319 to position 405 as set forth in SEQ ID NO: 19 derived from *Zea mays,* corresponds to an amino acid sequence from positions 107 to position 135 as set forth in SEQ ID NO: 20 derived from *Zea mays,* or is encoded by a nucleotide sequence which corresponds to nucleotides from position 319 to position 405 as set forth in SEQ ID NO: 16 derived from *Sorghum bicolor,* corresponds to an amino acid sequence from positions 107 to position 135 as set forth in SEQ ID NO: 17 derived from *Sorghum bicolor,* or having the consensus sequence of SEQ ID NO: 6, f) the loop2 which is encoded by a nucleotide sequence which corresponds to nucleotides from position 466 to position 486 as set forth in SEQ ID NO: 10 derived from *Arabidopsis thaliana,* corresponds to an amino acid sequence from positions 156 to position 162 as set forth in SEQ ID NO: 11 derived from *Arabidopsis thaliana,* or is encoded by a nucleotide sequence which corresponds to nucleotides from position 394 to position 414 as set forth in SEQ ID NO: 22 derived from *Beta vulgaris,* corresponds to an amino acid sequence from positions 132 to position 138 as set forth in SEQ ID NO: 23 derived from *Beta vulgaris,* or is encoded by a nucleotide sequence which corresponds to nucleotides from position 472 to position 492 as set forth in SEQ ID NO: 13 derived from *Brassica napus,* corresponds to an amino acid sequence from positions 158 to position 164 as set forth in SEQ ID NO: 14 derived from *Brassica napus,* or is encoded by a nucleotide sequence which corresponds to nucleotides from position 406 to position 426 as set forth in SEQ ID NO: 19 derived from *Zea mays,* corresponds to an amino acid sequence from positions 136 to position 142 as set forth in SEQ ID NO: 20 derived from *Zea mays,* or is encoded by a nucleotide sequence which corresponds to nucleotides from position 406 to position 426 as set forth in SEQ ID NO: 16 derived from *Sorghum bicolor,* corresponds to an amino acid sequence from positions 136 to position 142 as set forth in SEQ ID NO: 17 derived from *Sorghum bicolor,* or having the consensus sequence of SEQ ID NO: 7, g) the α3-helix which is encoded by a nucleotide sequence which corresponds to nucleotides from position 487 to position 516 as set forth in SEQ ID NO: 10 derived from *Arabidopsis thaliana,* corresponds to an amino acid sequence from positions 163 to position 172 as set forth in SEQ ID NO: 11 derived from *Arabidopsis thaliana,* or is encoded by a nucleotide sequence which corresponds to nucleotides from position 415 to position 444 as set forth in SEQ ID NO: 22 derived from *Beta vulgaris,* corresponds to an amino acid sequence from positions 139 to position 148 as set forth in SEQ ID NO: 23 derived from *Beta vulgaris,* or is encoded by a nucleotide sequence which corresponds to nucleotides from position 493 to position 522 as set forth in SEQ ID NO: 13 derived from *Brassica napus,* corresponds to an amino acid sequence from positions 165 to position 174 as set forth in SEQ ID NO: 14 derived from *Brassica napus,* or is encoded by a nucleotide sequence which corresponds to nucleotides from position 427 to position 456 as set forth in SEQ ID NO: 19 derived from *Zea mays,* corresponds to an amino acid sequence from positions 143 to position 152 as set forth in SEQ ID NO: 20 derived from *Zea mays,* or is encoded by a nucleotide sequence which corresponds to nucleotides from position 427 to position 456 as set forth in SEQ ID NO: 16 derived from *Sorghum bicolor,* corresponds to an amino acid sequence from positions 143 to position 152 as set forth in SEQ ID NO: 17 derived from *Sorghum bicolor,* or having the consensus sequence of SEQ ID NO: 8, or h) the C-terminal domain which is encoded by a nucleotide sequence which corresponds to nucleotides from position 517 to position 534 as set forth in SEQ ID NO: 10 derived from *Arabidopsis thaliana,* corresponds to an amino acid sequence from positions 173 to position 178 as set forth in SEQ ID NO: 11 derived from *Arabidopsis thaliana,* or is encoded by a nucleotide sequence which corresponds to nucleotides from position 445 to position 462 as set forth in SEQ ID NO: 22 derived from *Beta vulgaris,* corresponds to an amino acid sequence from positions 149 to position 154 as set forth in SEQ ID NO: 23 derived from *Beta vulgaris,* or is encoded by a nucleotide sequence which corresponds to nucleotides from position 523 to position 540 as set forth in SEQ ID NO: 13 derived from *Brassica napus,* corresponds to an amino acid sequence from positions 175 to position 180 as set forth in SEQ ID NO: 14 derived from *Brassica napus,* or is encoded by a nucleotide sequence which corresponds to nucleotides from position 457 to position 471 as set forth in SEQ ID NO: 19 derived from *Zea mays,* corresponds to an amino acid sequence from positions 153 to position 157 as set forth in SEQ ID NO: 20 derived from *Zea mays,* or is encoded by a nucleotide sequence which corresponds to nucleotides from position 457 to position 471 as set forth in SEQ ID NO: 16 derived from *Sorghum bicolor,* corresponds to an amino acid sequence from positions 153 to position 157 as set forth in SEQ ID NO: 17 derived from *Sorghum bicolor,* or having the consensus sequence of SEQ ID NO: 9. Part of α1-helix, the complete loop1 and complete α2-helix are positioned within the CATD domain of the CENH3 protein as defined above.

The non-mutated N-terminal tail domain of the CENH3 protein is partly conserved among plant species (see Figure). In the present disclosure, any amino acid position given with respect to these two conserved parts of the N-terminal tail domain (part A and part B) or the below described consensus sequence is referring to the following numbering system. Conserved part A and part B of the N-terminal tail domain can be separated by one or more amino acids. The specific number varies from plant species to plant species. For that in the consensus sequence a "*" has been introduced as place holder. Preferably, the non-mutated N-terminal tail domain exhibits the amino acid sequence as given in Table 1.

**Table 1: Specified amino acids in the N-terminal tail domain of the CENH3 protein**

| Conserved part - position within the N-terminal | Amino acid(s) |
|---|---|
| A / 1 | M |
| A / 2 | A |
| A / 3 | R |
| A / 4 | T, V, I or A |
| A / 5 | K or R |
| A / 6 | H, T, Q or K |
| A / 7 | X |
| A / 8 | X |
| A / 9 | V, A, P, G, N, P, R, S or H |
| A / 10 | T, R, S, L, K, H, N, A or P |
| A / 11 | R, K, A, N or T |
| A / 12 | S, A, T, L, K, R, D, N or E |
| A / 13 | Q, T, R, A, P, S, G, N, V, K or R |
| A / 14 | P, T, D, E, Q, S, N, G, A, K or R |
| A / 15 | R, N, H,V, G, K, S, A, T, E or P |
| B / 1 | R, D, K, V, G, P, S, Q, T or A |
| B / 2 | G, A, S, K, R, V, T, P or Q |
| B / 3 | S, T, K, V, R, Q, A, E, G, P or D |
| B / 4 | Q, P, N, T, E, K, G, S, R, A or D |
| B / 5 | K, Q, P, G, N, T, H or R |
| B / 6 | X |
| B / 7 | K, R, Q or H |
| B / 8 | K, Q or R |
| B / 9 | S, A, T, K, P or R |
| B / 10 | Y, F, H, T, K, R, F or Q |
| B / 11 | R |
| B / 12 | Y, R, W, F, L, N or S |
| B / 13 | R or K |
| B / 14 | P, A or S |

More preferably, the N terminal tail domain has the consensus sequences of SEQ ID NO: 1 (part A, before *) and SEQ ID NO: 2 (part B, beyond *), which is

MARTK HXXAR RSRKR * QSQTQ XKKKH RYRP.
5 10 15 5 10 14

As indicated above, the N-terminal tail domain comprises unspecified [marked as X] and specified amino acids [marked as one letter code]. Instead of an unspecified amino acid the "X" can also be a gap of at least one amino acid.

The non-mutated aN-helix of the CENH3 protein is highly conserved among plant species and is 15 amino acids long starting with position 1 and ending with position 15. In the present disclosure, any amino acid position given with respect to the aN-helix or the below described consensus sequence of SEQ ID NO: 3 is referring to this numbering system. Preferably, the non-mutated aN-helix exhibits the amino acid sequence as given in Table 2.

**Table 2: Specified amino acids in the aN-helix of the CENH3 protein**

| Position within the aN-helix | Amino acid(s) |
|---|---|
| 1 | G |
| 2 | T |
| 3 | V |
| 4 | A |
| 5 | L |
| 6 | K, W or R |
| 7 | E or Q |
| 8 | I |
| 9 | R |
| 10 | X |
| 11 | F, Y or L |
| 12 | Q or R |
| 13 | K |
| 14 | Q, S or T |
| 15 | T, F, W, V, C or A |

More preferably, the aN-helix has the consensus sequence of SEQ ID NO: 3, which is

GTVAL REIRX FQKTT.
5 10 15

As indicated above, the aN-helix comprises unspecified [marked as X] and specified amino acids [marked as one letter code].

The non-mutated α1-helix of the CENH3 protein is conserved among plant species and is 11 amino acids long starting with position 1 and ending with position 11. In the present disclosure, any amino acid position given with respect to the α1-helix or the below described consensus sequence of SEQ ID NO: 4 is referring to this numbering system. Preferably, the non-mutated α1-helix exhibits the amino acid sequence as given in Table 3.

**Table 3: Specified amino acids in the α1-helix of the CENH3 protein**

| Position within the α1-helix | Amino acid(s) |
|---|---|
| 1 | A, F, R or S |
| 2 | A, M or S |
| 3 | S, P, T, A or C |
| 4 | F |
| 5 | I, V, M, L, S or A |
| 6 | R |
| 7 | E, T, V, L, C, Q or A |
| 8 | V or I |
| 9 | R or K |
| 10 | S, E, M, T, E, Q, G or D |
| 11 | I, V, L or T |

More preferably, the α1-helix has the consensus sequence of SEQ ID NO: 4, which is

AAPFI RLVRE I.
5 10

As indicated above, the α1-helix comprises specified amino acids [marked as one letter code].

The non-mutated loop1 of the CENH3 protein is highly conserved among plant species and is 13 amino acids long starting with position 1 and ending with position 13. In the present disclosure, any amino acid position given with respect to the loop1 or the below described consensus sequence of SEQ ID NO: 5 is referring to this numbering system. Preferably, the non-mutated loop1 exhibits the amino acid sequence as given in Table 4.

**Table 4: Specified amino acids in the loop1 of the CENH3 protein**

| Position within the loop1 | Amino acid(s) |
|---|---|
| 1 | T, S or A |
| 2 | H, Q, N, A, Y, F, G, D or E |
| 3 | M, Q, I, F, Y, A, E, N, R, L, H or G |
| 4 | L, F, V, I or Y |
| 5 | A, T, S, C or M |
| 6 | P, N, D, R, A, T, F, R, H, S or K |
| 7 | X |
| 8 | Q, Y, D, K, R, E, G, S, P, H, N or A |
| 9 | I, V or P |
| 10 | N, G, T, E, or S |
| 11 | R or P |
| 12 | W or Y |
| 13 | T, Q or S |

More preferably, the loop1 has the consensus sequence of SEQ ID NO: 5, which is

TNFLA PXEVT RWT.
5 10 13

As indicated above, the loop1 comprises unspecified [marked as X] and specified amino acids [marked as one letter code].

The non-mutated α2-helix of the CENH3 protein is highly conserved among plant species and is 29 amino acids long starting with position 1 and ending with position 29. In the present disclosure, any amino acid position given with respect to the α2-helix or the below described consensus sequence of SEQ ID NO: 6 is referring to this numbering system. Preferably, the non-mutated α2-helix exhibits the amino acid sequence as given in Table 5.

**Table 5: Specified amino acids in the α2-helix of the CENH3 protein**

| Position within the α2-helix | Amino acid(s) |
|---|---|
| 1 | A, P, V or L |
| 2 | E, D, Q, H or L |
| 3 | A |
| 4 | L or V |
| 5 | V, L, M, I, R, Y or T |
| 6 | S or A |
| 7 | I or L |
| 8 | Q |
| 9 | E |
| 10 | A or S |
| 11 | A or T |
| 12 | E |
| 13 | D, N, F, I or Y |
| 14 | Y, F or H |
| 15 | L, I or V |
| 16 | V or I |
| 17 | G, R, E, H, N, T, E, D or Q |
| 18 | L, M or I |
| 19 | F, M or L |
| 20 | S, E, D or G |
| 21 | D, M, V, N, E, A, R or K |
| 22 | S, G, A or T |
| 23 | M, W, N or H |
| 24 | L or H |
| 25 | C or L |
| 26 | A or T |
| 27 | L or I |
| 28 | H |
| 29 | A or S |

More preferably, the α2-helix has the consensus sequence of SEQ ID NO: 6, which is

AEALL ALQEA AEDFL VHLFE DAMLC AIHA.
5 10 15 20 25 29

As indicated above, the α2-helix comprises specified amino acids [marked as one letter code].

The non-mutated loop2 of the CENH3 protein is highly conserved among plant species and is 7 amino acids long starting with position 1 and ending with position 7. In the present disclosure, any amino acid position given with respect to the loop2 or the below described consensus sequence of SEQ ID NO: 7 is referring to this numbering system. Preferably, the non-mutated loop2 exhibits the amino acid sequence as given in Table 6.

**Table 6: Specified amino acids in the loop2 of the CENH3 protein**

| Position within the loop2 | Amino acid(s) |
|---|---|
| 1 | R, K or H |
| 2 | R |
| 3 | V or I |
| 4 | T |
| 5 | L, I or V |
| 6 | M or L |
| 7 | R, K, Q, L or T |

More preferably, the loop2 has the consensus sequence of SEQ ID NO: 7, which is

KRVTL MK.
5 7

As indicated above, the loop2 comprises specified amino acids [marked as one letter code].

The non-mutated α3-helix of the CENH3 protein is highly conserved among plant species and is 10 amino acids long starting with position 1 and ending with position 10. In the present disclosure, any amino acid position given with respect to the α3-helix or the below described consensus sequence of SEQ ID NO: 8 is referring to this numbering system. Preferably, the non-mutated α3-helix exhibits the amino acid sequence as given in Table 7.

**Table 7: Specified amino acids in the α3-helix of the CENH3 protein**

| Position within the α3-helix | Amino acid(s) |
|---|---|
| 1 | K or R |
| 2 | D |
| 3 | F, L, I, M or W |
| 4 | E, Q or R |
| 5 | L |
| 6 | A or T |
| 7 | R |
| 8 | R |
| 9 | L or I |
| 10 | G, R or T |

More preferably, the α3-helix has the consensus sequence of SEQ ID NO: 8, which is

KDFEL ARRLG.
5 10

As indicated above, the α3-helix comprises specified amino acids [marked as one letter code].

The non-mutated C-terminal domain of the CENH3 protein varies in length. Under consideration of numerous plant species (see below) we identified length of up to 7 amino acids. According to the present disclosure, any amino acid position given with respect to the C-terminal domain or the below described consensus sequence of SEQ ID NO: 9 is referring to this numbering system. Preferably, the non-mutated C-terminal domain exhibits the amino acid sequence as given in Table 8.

**Table 8: Specified amino acids in the C-terminal domain of the CENH3 protein**

| Position within the C-terminal domain | Amino acid(s) |
|---|---|
| 1 | G, K, A, S or T |
| 2 | K, R, I or A |
| 3 | G, E or A |
| 4 | R, Q or V |
| 5 | P, G, I, Q, L, S or H |
| 6 | W, L, F or V |
| 7 | X |

More preferably, the C-terminal domain has the consensus sequence of SEQ ID NO: 9, which is

GKGRP W.
5 6

As indicated above, the C-terminal domain comprises specified amino acids [marked as one letter code].

According to the present disclosure, a mutation causing an alteration of any of the unspecified or the specified amino acid as defined in Table 1 or in SEQ ID NO: 1 or 2, or in Table 2 or in SEQ ID NO: 3, or in Table 3 or in SEQ ID NO: 4, or in Table 4 or in SEQ ID NO: 5, or in Table 5 or in SEQ ID NO: 6, or in Table 6 or in SEQ ID NO: 7, in Table 7 or in SEQ ID NO: 8, or in Table 8 or in SEQ ID NO: 9, preferably a substitution or deletion of the amino acid(s), can produce the desired plant possessing the capability to produce haploid progeny.

An unspecified amino acid as given in Table 1 or in SEQ ID NO: 1 or 2, or in Table 2 or in SEQ ID NO: 3, or in Table 3 or in SEQ ID NO: 4, or in Table 4 or in SEQ ID NO: 5, or in Table 5 or in SEQ ID NO: 6, or in Table 6 or in SEQ ID NO: 7, in Table 7 or in SEQ ID NO: 8, or in Table 8 or in SEQ ID NO: 9 is an amino acid which although being specified in a group of particular plant species, in a particular plant genus or in a particular plant species is not conserved in a greater range of plant species. Thus, an unspecified amino acid of SEQ ID NO: 1, 2, 3, 4, 5, 6, 7, 8 or 9 or as given in Table 1, Table 2, Table 3, Table 4, Table 5, Table 6, Table 7 or Table 8 is in a group of particular plant species, in a particular plant genus or in a particular plant species a well-defined, specific amino acid, which, however, is possibly not found at the same place in another plant species. Thus, an amino acid substitution of an unspecified amino acid of SEQ ID NO: 1 or as indicated in Table 1 means that in a plant, namely in a specific plant species, the specific but not conserved amino acid is substituted by another amino acid than naturally occurring at that place in this group of particular plant species, in this particular plant genus or in this particular plant species in the endogenously coded native CENH3 protein of said plant species. Furthermore, an unspecified amino acid as well as a specified amino acid can be essential with respect to processes of protein folding or protein stability. The alteration of such amino acid can lead to a mutant CENH3 having impaired stability or an incorrect folding.

Specified amino acids given in Table 1, Table 2, Table 3, Table 4, Table 5, Table 6, Table 7 or Table 8 and in particular specified amino acids of SEQ ID NO: 1, 2, 3, 4, 5, 6, 7, 8 or 9 are those which occur in a broad range of plant species, preferably such as listed below, and which are thus well conserved.

In the context of the present disclosure, the consensus sequence of SEQ ID NO: 1, 2, 3, 4, 5, 6, 7, 8 and 9 has been compiled from the sequences of the protein segments derived from species selected from the group consisting of *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas,Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Astragalus sinicus, Lotus japonicas, Torenia fournieri, Allium cepa, Allium fistulosum, Allium sativum,* and *Allium tuberosum.*

As further disclosed but not claimed, the at least one mutation causes a substitution of a specified amino acid as defined in Table 1, Table 2, Table 3, Table 4, Table 5, Table 6, Table 7 or Table 8. Thus, the plant according to the present disclosure comprises at least one substitution of the specified amino acids as defined in Table 1, Table 2, Table 3, Table 4, Table 5, Table 6, Table 7 or Table 8, i.e. those amino acids which are conserved and named in Table 1, Table 2, Table 3, Table 4, Table 5, Table 6, Table 7 or Table 8. The substitution of a specified amino acid as defined in Table 1 shall mean the substitution of an amino acid selected from the group consisting of:
a) methionine at position 1 of part A,
b) alanine at position 2 of part A,
c) arginine at position 3 of part A,
d) threonine, valine, isoleucine or alanine at position 4 of part A,
e) lysine or arginine at position 5 of part A,
f) histidine, threonine, glutamine or lysine at position 6 of part A,
g) valine, alanine, proline, glycine, asparagine, proline, arginine, serine or histidine at position 9 of part A,
h) threonine, arginine, serine, leucine, lysine, histidine, asparagine, alanine or proline at position 10 of part A,
i) arginine, lysine, alanine, asparagine or threonine at position 11 of part A,
j) serine, alanine, threonine, leucine, lysine, arginine, aspartic acid, asparagine or glutamic acid at position 12 of part A,
k) glutamine, threonine, arginine, alanine, proline, serine, glycine, asparagine, valine, lysine or arginine at position 13 of part A,
l) proline, threonine, aspartic acid, glutamic acid, glutamine, serine, asparagine, glycine, alanine, lysine, arginine at position 14 of part A, and
m) arginine, asparagine, histidine, valine, glycine, lysine, serine, alanine, threonine, glutamic acid, proline at position 15 of part A;
n) arginine, aspartic acid, lysine, valine, glycine, proline, serine, glutamine, threonine or alanine at position 1 of part B,
o) glycine, alanine, serine, lysine, arginine, valine, threonine, proline or glutamine at position 2 of part B,
p) serine, threonine, lysine, valine, arginine, glutamine, alanine, glutamic acid, glycine, proline und aspartic acid at position 3 of part B,
q) glutamine, proline, asparagine, threonine, glutamic acid, lysine, glycine, serine, arginine, alanine or aspartic acid at position 4 of part B,
r) lysine, glutamine, proline, glycine, asparagine, threonine, histidine or arginine at position 5 of part B,
s) lysine, arginine, glutamine or histidine at position 7 of part B,
t) lysine, glutamine or arginine at position 8 of part B,
u) serine, alanine, threonine, lysine, proline or arginine at position 9 of part B,
v) tyrosine, phenylalanine, histidine, threonine, lysine, arginine, phenylalanine or glutamine at position 10 of part B,
w) arginine at position 11 of part B,
x) tyrosine, arginine, tryptophan, phenylalanine, leucine, asparagine or serine at position 12 of part B,
y) arginine or lysine at position 13 of part B, and
z) proline, alanine or serine at position 14 of part B.

The substitution of a specified amino acid as defined in Table 2 shall mean the substitution of an amino acid selected from the group consisting of:
a) glycine at position 1,
b) threonine at position 2,
c) valine at position 3,
d) alanine at position 4,
e) leucine at position 5,
f) lysine, tryptophan or arginine at position 6,
g) glutamic acid or glutamine at position 7,
h) isoleucine at position 8,
i) arginine at position 9,
j) phenylalanine, tyrosine or leucine at position 11,
k) glutamine or arginine at position 12,
l) lysine at position 13,
m) glutamine, serine or threonine at position 14, and
n) threonine, phenylalanine, tryptophan, valine, cysteine or alanine at position 15.

The substitution of a specified amino acid as defined in Table 3 shall mean the substitution of an amino acid selected from the group consisting of:
a) alanine, phenylalanine, arginine or serine at position 1,
b) alanine, methionine or serine at position 2,
c) serine, proline, threonine, alanine or cysteine at position 3,
d) phenylalanine at position 4,
e) isoleucine, valine, methionine, leucine, serine or alanine at position 5,
f) arginine at position 6,
g) glutamic acid, threonine, valine, leucine, cysteine, glutamine or alanine at position 7,
h) valine or isoleucine at position 8,
i) arginine or lysine at position 9,
j) serine, glutamic acid, methionine, threonine, glutamic acid, glutamine, glycine or aspartic acid at position 10, and
k) isoleucine, valine, leucine or threonine at position 11.

The substitution of a specified amino acid as defined in Table 4 shall mean the substitution of an amino acid selected from the group consisting of:
a) threonine, serine or alanine at position 1,
b) histidine, glutamine, asparagine, alanine, tyrosine, phenylalanine, glycine, aspartic acid or glutamic acid at position 2,
c) methionine, glutamine, isoleucine, phenylalanine, tyrosine, alanine, glutamic acid, asparagine, arginine, leucine, histidine or glycine at position 3,
d) leucine, phenylalanine, valine, isoleucine or tyrosine at position 4,
e) alanine, threonine, serine, cysteine or methionine at position 5,
f) proline, asparagine, aspartic acid, arginine, alanine, threonine, phenylalanine, arginine, histidine, serine or lysine at position 6,
g) glutamine, tyrosine, aspartic acid, lysine, arginine, glutamic acid, glycine, serine, proline, histidine, asparagine or alanine at position 8,
h) isoleucine, valine or proline at position 9,
i) asparagine, glycine, threonine, glutamic acid or serine at position 10,
j) arginine or proline at position 11,
k) tryptophan or tyrosine at position 12, and
l) threonine, glutamine or serine at position 13.

The substitution of a specified amino acid as defined in Table 5 shall mean the substitution of an amino acid selected from the group consisting of:
a) alanine, proline, valine or leucine at position 1,
b) glutamic acid, aspartic acid, glutamine, histidine or leucine at position 2,
c) alanine at position 3,
d) leucine or valine at position 4,
e) valine, leucine, methionine, isoleucine, arginine, tyrosine or threonine at position 5,
f) serine or alanine at position 6,
g) isoleucine or leucine at position 7,
h) glutamine at position 8,
i) glutamic acid at position 9,
j) alanine or serine at position 10,
k) alanine or threonine at position 11,
l) glutamic acid at position 12,
m) aspartic acid, asparagine, phenylalanine, isoleucine or tyrosine at position 13,
n) tyrosine, phenylalanine or histidine at position 14,
o) leucine, isoleucine or valine at position 15,
p) valine or isoleucine at position 16,
q) glycine, arginine, glutamic acid, histidine, asparagine, threonine, glutamic acid, aspartic acid or glutamine at position 17,
r) leucine, methionine or isoleucine at position 18,
s) phenylalanine, methionine or leucine at position 19,
t) serine, glutamic acid, aspartic acid or glycine at position 20,
u) aspartic acid, methionine, valine, asparagine, glutamic acid, alanine, arginine, lysine at position 21,
v) serine, glycine, alanine or threonine at position 22,
w) methionine, tryptophan, asparagine or histidine at position 23,
x) leucine or histidine at position 24,
y) cysteine or leucine at position 25,
z) alanine or threonine at position 26,
aa) leucine or isoleucine at position 27,
bb) histidine at position 28, and
cc) alanine or serine at position 29.

The substitution of a specified amino acid as defined in Table 6 shall mean the substitution of an amino acid selected from the group consisting of:
a) arginine, lysine or histidine at position 1,
b) arginine at position 2,
c) valine or isoleucine at position 3,
d) threonine at position 4,
e) leucine, isoleucine or valine at position 5,
f) methionine or leucine at position 6, and
g) arginine, lysine, glutamine, leucine or threonine at position 7.

The substitution of a specified amino acid as defined in Table 7 shall mean the substitution of an amino acid selected from the group consisting of:
a) lysine or arginine at position 1,
b) aspartic acid at position 2,
c) phenylalanine, leucine, isoleucine, methionine or tryptophan at position 3,
d) glutamic acid, glutamine or arginine at position 4,
e) leucine at position 5,
f) alanine or threonine at position 6,
g) arginine at position 7,
h) arginine at position 8,
i) leucine or isoleucine at position 9, and
j) glycine, arginine or threonine at position 10.

The substitution of a specified amino acid as defined in Table 8 shall mean the substitution of an amino acid selected from the group consisting of:
a) glycine, lysine, alanine, serine or threonine at position 1,
b) lysine, arginine, isoleucine or alanine at position 2,
c) glycine, glutamic acid or alanine at position 3,
d) arginine, glutamine or valine at position 4,
e) proline, glycine, isoleucine, glutamine, leucine, serine or histidine at position 5, and
f) tryptophan, leucine, phenylalanine or valine at position 6.

As further disclosed but not claimed, the at least one mutation causes a substitution of a specified amino acid of SEQ ID NO: 1. Thus, the plant according to the disclosure comprises at least one substitution of the specified amino acids of SEQ ID NO: 1, i.e. those amino acids which are highly conserved and named in the consensus sequence of SEQ ID NO: 1. The substitution of a specified amino acid of SEQ ID NO: 1 shall mean the substitution of an amino acid selected from group consisting of:
a) methionine at position 1,
b) alanine at position 2,
c) arginine at position 3,
d) threonine at position 4,
e) lysine at position 5,
f) histidine at position 6,
g) alanine at position 9,
h) arginine at position 10,
i) arginine at position 11,
j) serine at position 12,
k) arginine at position 13,
l) lysine at position 14, and
m) arginine at position 15.

As further disclosed but not claimed, the at least one mutation causes a substitution of a specified amino acid of SEQ ID NO: 2. Thus, the plant according to the disclosure comprises at least one substitution of the specified amino acids of SEQ ID NO: 2, i.e. those amino acids which are highly conserved and named in the consensus sequence of SEQ ID NO: 2. The substitution of a specified amino acid of SEQ ID NO: 2 shall mean the substitution of an amino acid selected from group consisting of:
a) glutamine at position 1,
b) serine at position 2,
c) glutamine at position 3,
d) threonine at position 4,
e) glutamine at position 5,
f) lysine at position 7,
g) lysine at position 8,
h) lysine at position 9,
i) histidine at position 10,
j) arginine at position 11,
k) tyrosine at position 12,
l) arginine at position 13, and
m) proline at position 14.

As further disclosed but not claimed, the at least one mutation causes a substitution of a specified amino acid of SEQ ID NO: 3. Thus, the plant according to the present disclosure comprises at least one substitution of the specified amino acids of SEQ ID NO: 3, i.e. those amino acids which are highly conserved and named in the consensus sequence of SEQ ID NO: 3. The substitution of a specified amino acid of SEQ ID NO: 3 shall mean the substitution of an amino acid selected from group consisting of:
a) glycine at position 1,
b) threonine at position 2,
c) valine at position 3,
d) alanine at position 4,
e) leucine at position 5,
f) arginine at position 6,
g) glutamic acid at position 7,
h) isoleucine at position 8,
i) arginine at position 9,
j) phenylalanine at position 11,
k) glutamine or arginine at position 12,
l) lysine at position 13,
m) threonine at position 14, and
n) threonine at position 15.

As further disclosed but not claimed, the at least one mutation causes a substitution of a specified amino acid of SEQ ID NO: 4. Thus, the plant according to the present disclosure comprises at least one substitution of the specified amino acids of SEQ ID NO: 4, i.e. those amino acids which are highly conserved and named in the consensus sequence of SEQ ID NO: 4. The substitution of a specified amino acid of SEQ ID NO: 4 shall mean the substitution of an amino acid selected from group consisting of:
a) alanine at position 1,
b) alanine at position 2,
c) proline at position 3,
d) phenylalanine at position 4,
e) isoleucine at position 5,
f) arginine at position 6,
g) leucine acid at position 7,
h) valine at position 8,
i) arginine at position 9,
j) glutamic acid at position 10, and
k) isoleucine at position 11.

As further disclosed but not claimed, the at least one mutation causes a substitution of a specified amino acid of SEQ ID NO: 5. Thus, the plant according to the present disclosure comprise at least one substitution of the specified amino acids of SEQ ID NO: 5, i.e. those amino acids which are highly conserved and named in the consensus sequence of SEQ ID NO: 5. The substitution of a specified amino acid of SEQ ID NO: 5 shall mean the substitution of an amino acid selected from group consisting of:
a) threonine at position 1,
b) asparagine at position 2,
c) phenylalanine at position 3,
d) leucine at position 4,
e) alanine at position 5,
f) proline at position 6,
g) glutamic acid at position 8,
h) valine at position 9,
i) threonine at position 10,
j) arginine at position 11,
k) tryptophan at position 12, and
l) threonine at position 13.

As further disclosed but not claimed, the at least one mutation causes a substitution of a specified amino acid of SEQ ID NO: 6. Thus, the plant according to the present disclosure comprises at least one substitution of the specified amino acids of SEQ ID NO: 6, i.e. those amino acids which are highly conserved and named in the consensus sequence of SEQ ID NO: 6. The substitution of a specified amino acid of SEQ ID NO: 6 shall mean the substitution of an amino acid selected from group consisting of:
a) alanine at position 1,
b) glutamic acid at position 2,
c) alanine at position 3,
d) leucine at position 4,
e) leucine at position 5,
f) alanine at position 6,
g) leucine at position 7,
h) glutamine at position 8,
i) glutamic acid at position 9,
j) alanine at position 10,
k) alanine at position 11,
I) glutamic acid at position 12,
m) aspartic acid at position 13,
n) phenylalanine at position 14,
o) leucine at position 15,
p) valine at position 16,
q) histidine at position 17,
r) leucine at position 18,
s) phenylalanine at position 19,
t) glutamic acid at position 20,
u) aspartic acid at position 21,
v) alanine at position 22,
w) methionine at position 23,
x) leucine at position 24,
y) cysteine at position 25,
z) alanine at position 26,
aa) isoleucine at position 27,
bb) histidine at position 28, and
cc) alanine at position 29.

As further disclosed but not claimed, the at least one mutation causes a substitution of a specified amino acid of SEQ ID NO: 7. Thus, the plant according to the present disclosure comprises at least one substitution of the specified amino acids of SEQ ID NO: 7, i.e. those amino acids which are highly conserved and named in the consensus sequence of SEQ ID NO: 7. The substitution of a specified amino acid of SEQ ID NO: 7 shall mean the substitution of an amino acid selected from group consisting of:
a) lysine at position 1,
b) arginine at position 2,
c) valine at position 3,
d) threonine at position 4,
e) leucine at position 5,
f) methionine at position 6, and
g) lysine at position 7.

As further disclosed but not claimed, the at least one mutation causes a substitution of a specified amino acid of SEQ ID NO: 8. Thus, the plant according to the present disclosure comprises at least one substitution of the specified amino acids of SEQ ID NO: 8, i.e. those amino acids which are highly conserved and named in the consensus sequence of SEQ ID NO: 8. The substitution of a specified amino acid of SEQ ID NO: 8 shall mean the substitution of an amino acid selected from group consisting of:
a) lysine at position 1,
b) aspartic acid at position 2,
c) phenylalanine at position 3,
d) glutamic acid at position 4,
e) leucine at position 5,
f) alanine at position 6,
g) arginine at position 7,
h) arginine at position 8,
i) leucine at position 9, and
j) glycine at position 10.

As further disclosed but not claimed, the at least one mutation causes a substitution of a specified amino acid of SEQ ID NO: 9. Thus, the plant according to the disclosure comprises at least one substitution of the specified amino acids of SEQ ID NO: 9, i.e. those amino acids which are highly conserved and named in the consensus sequence of SEQ ID NO: 9. The substitution of a specified amino acid of SEQ ID NO: 9 shall mean the substitution of an amino acid selected from group consisting of:
a) glycine at position 1,
b) lysine at position 2,
c) glycine at position 3,
d) arginine acid at position 4,
e) proline at position 5, and
f) tryptophan at position 6.

As further disclosed but not claimed, the at least one mutation causes a substitution of a specified amino acid in the N-terminal tail domain, wherein the amino acid arginine at position 3 of SEQ ID NO: 1 is substituted, preferably for lysine, or the amino acid arginine at position 2 of SEQ ID NO: 23 is substituted, preferably for lysine, or the amino acid arginine at position 10 of SEQ ID NO: 1 is substituted, preferably for phenylalanine, or the amino acid serine at position 9 of SEQ ID NO: 14 is substituted, preferably for phenylalanine, or the amino acid arginine at position 16 of SEQ ID NO: 14 is substituted, preferably for glutamine, or the amino acid serine at position 24 of SEQ ID NO: 14 is substituted, preferably for leucine, or the amino acid serine at position 24 of SEQ ID NO: 14 is substituted, preferably for leucine, or the amino acid alanine at position 25 of SEQ ID NO: 17 is substituted, preferably for threonine, or the amino acid glutamic acid at position 29 of SEQ ID NO: 14 is substituted, preferably for lysine, or the amino acid glycine at position 30 of SEQ ID NO: 14 is substituted, preferably for aspartic acid, or the amino acid alanine at position 33 of SEQ ID NO: 14 or at position 32 of SEQ ID NO: 20 is substituted, preferably for threonine, or the amino acid proline at position 35 of SEQ ID NO: 14 is substituted, preferably for leucine, or the amino acid glutamic acid at position 35 of SEQ ID NO: 20 is substituted, preferably for lysine, or the amino acid serine at position 41 of SEQ ID NO: 14 is substituted, preferably for asparagine, or the amino acid glycine at position 43 of SEQ ID NO: 14 is substituted, preferably for glutamic acid, or the amino acid proline at position 50 of SEQ ID NO: 14 is substituted, preferably for serine, or the amino acid proline at position 55 of SEQ ID NO: 14 is substituted, preferably for leucine, or the amino acid glycine at position 57 of SEQ ID NO: 14 is substituted, preferably for aspartic acid, or the amino acid glycine at position 61 of SEQ ID NO: 14 is substituted, preferably for glutamic acid, or the amino acid arginine at position 65 of SEQ ID NO: 14 is substituted, preferably for glutamine, or the amino acid arginine at position 65 of SEQ ID NO: 14 is substituted, preferably for stop signal, or the amino acid proline at position 71 of SEQ ID NO: 14 is substituted, preferably for serine, or the amino acid aspartic acid at position 46 of SEQ ID NO: 23 is substituted, preferably for asparagine or glycine, or the amino acid lysine at position 7 of SEQ ID NO: 2 is substituted, preferably for serine, or the amino acid proline at position 56 of SEQ ID NO: 20 is substituted, preferably for serine, or the amino acid proline at position 14 of SEQ ID NO: 2 is substituted, preferably for valine, or the amino acid alanine at position 62 of SEQ ID NO: 17 is substituted, preferably for valine.

As further disclosed but not claimed, the at least one mutation causes a substitution of a specified amino acid in the aN-helix, wherein the amino acid threonine at position 2 of SEQ ID NO: 3 is substituted, preferably for serine, or the amino acid threonine at position 64 of SEQ ID NO: 17 is substituted, preferably for serine.

As further disclosed but not claimed, the at least one mutation causes a substitution of a specified amino acid in the α1-helix, wherein the amino acid alanine at position 1 of SEQ ID NO: 4 is substituted, preferably for threonine, or the amino acid alanine at position 105 of SEQ ID NO: 14 is substituted, preferably for threonine, or the amino acid arginine at position 6 of SEQ ID NO: 4 is substituted, preferably for glutamine, or the amino acid arginine at position 110 of SEQ ID NO: 14 is substituted, preferably for glutamine, or the amino acid valine at position 89 of SEQ ID NO: 20 is substituted, preferably for methionine, or the amino acid glutamic acid at position 10 of SEQ ID NO: 4 is substituted, preferably for asparagine, or the amino acid serine at position 114 of SEQ ID NO: 14 is substituted, preferably for asparagine.

As further disclosed but not claimed, the at least one mutation causes a substitution of a specified amino acid in the loop1, wherein the amino acid asparagine at position 2 of SEQ ID NO: 5 is substituted, preferably for valine, or the amino acid alanine at position 95 of SEQ ID NO: 17 is substituted, preferably for valine, or the amino acid proline at position 6 of SEQ ID NO: 5 is substituted, preferably for serine, or the amino acid proline at position 121 of SEQ ID NO: 14 is substituted, preferably for serine, or the amino acid tryptophan at position 12 of SEQ ID NO: 5 is substituted, preferably for a stop signal, or the amino acid tryptophan at position 127 of SEQ ID NO: 14 is substituted, preferably for a stop signal.

As further disclosed but not claimed, the at least one mutation causes a substitution of a specified amino acid in the α2-helix, wherein the amino acid alanine at position 1 of SEQ ID NO: 6 is substituted, preferably for threonine, or the amino acid alanine at position 107 of SEQ ID NO: 20 is substituted, preferably for threonine, or the amino acid leucine at position 4 of SEQ ID NO: 6 is substituted, preferably for phenylalanine or glutamine, or the amino acid leucine at position 132 of SEQ ID NO: 14 or position 106 of SEQ ID NO: 23 is substituted, preferably for phenylalanine or glutamine, or the amino leucine at position 7 of SEQ ID NO: 6 is substituted, preferably for proline, or the amino acid leucine at position 109 of SEQ ID NO: 23 is substituted, preferably for proline, or the amino acid glutamine at position 8 of SEQ ID NO: 6 is substituted, preferably for a stop signal or leucine, or the amino acid glutamine at position 114 of SEQ ID NO: 20 or position 110 of SEQ ID NO: 23 is substituted, preferably for a stop signal or leucine, or the amino acid alanine at position 10 of SEQ ID NO: 6 is substituted, preferably for threonine, or the amino acid alanine at position 138 of SEQ ID NO: 14 is substituted, preferably for threonine, or the amino acid cysteine at position 25 of SEQ ID NO: 6 is substituted, preferably for tyrosine, or the amino acid cysteine at position 153 of SEQ ID NO: 14 is substituted, preferably for tyrosine, or the amino acid alanine at position 26 of SEQ ID NO: 6 is substituted, preferably for valine, or the amino acid alanine at position 154 of SEQ ID NO: 14 is substituted, preferably for valine.

According to the present invention, the at least one mutation causes a substitution of a specified amino acid in the loop2, wherein the amino acid arginine at position 159 of SEQ ID NO: 14 is substituted for histidine, or the amino acid valine at position 160 of SEQ ID NO: 14 is substituted for isoleucine, or the amino acid threonine at position 139 of SEQ ID NO: 20 is substituted for isoleucine.

As further disclosed but not claimed, the at least one mutation may cause a substitution of a specified amino acid in the loop2, wherein the amino acid arginine at position 2 of SEQ ID NO: 7 may be substituted, preferably for histidine, or the amino acid valine at position 3 of SEQ ID NO: 7 may be substituted, preferably for isoleucine, or the amino acid threonine at position 4 of SEQ ID NO: 7 may be substituted.

As further disclosed but not claimed, the at least one mutation causes a substitution of a specified amino acid in the α3-helix, wherein the amino acid aspartic acid at position 2 of SEQ ID NO: 8 is substituted, preferably for asparagine, or the amino acid aspartic acid at position 166 of SEQ ID NO: 14 is substituted, preferably for asparagine, or the amino acid glutamic acid at position 4 of SEQ ID NO: 8 is substituted, preferably for lysine, or the amino acid glutamic acid at position 168 of SEQ ID NO: 14 is substituted, preferably for lysine, or the amino acid arginine at position 8 of SEQ ID NO: 8 is substituted, preferably for histidine, or the amino acid arginine at position 172 of SEQ ID NO: 14 is substituted, preferably for histidine, or the amino acid leucine at position 9 of SEQ ID NO: 8 is substituted, preferably for phenylalanine, or the amino acid leucine at position 173 of SEQ ID NO: 14 is substituted, preferably for phenylalanine, or the amino acid glycine at position 10 of SEQ ID NO: 8 is substituted, preferably for glutamic acid, or the amino acid glycine at position 174 of SEQ ID NO: 14 or position 152 of SEQ ID NO: 20 is substituted, preferably for glutamic acid.

As further disclosed but not claimed, the at least one mutation causes a substitution of a specified amino acid in the C-terminal domain, wherein the amino acid glycine at position 3 of SEQ ID NO: 9 is substituted, preferably for histidine, or the amino acid arginine at position 155 of SEQ ID NO: 20 is substituted, preferably for histidine, or the amino acid arginine at position 4 of SEQ ID NO: 9 is substituted, preferably for lysine, or the amino acid arginine at position 178 of SEQ ID NO: 14 is substituted, preferably for lysine, or the amino acid serine at position 157 of SEQ ID NO: 17 is substituted, preferably for leucine.

As further disclosed but not claimed, the at least one mutation is positioned in a splicing site of the genomic nucleotide sequence encoding the CENH3 protein and/or the at least one mutation creates a new splicing site within an exon. Preferably, a plant which is heterozygous for such mutation(s) is viable. Such mutation(s) can cause a malfunctioning splicing site (splicing error), which then results in an increased cellular translational production of non-fully functional CENH3 proteins, which show e.g. an impaired stability, a reduced binding affinity to DNA, a changed geometric shape of the protein, preferably a changed secondary or tertiary structure, or a disordered protein folding compared to the fully functional wildtype CENH3 protein.

As further disclosed but not claimed, the at least one mutation causes a splicing error, preferably in intron 1 of SEQ ID NO: 12, leading to an alteration of the amino acid sequence of the CENH3 protein of *Brassica napus* after amino acid at position 18 of SEQ ID NO: 14, a splicing error, preferably in intron 2 of SEQ ID NO: 12, leading to an alteration of the amino acid sequence of the CENH3 protein of *Brassica napus* after amino acid at position 33 of SEQ ID NO: 14, a splicing error, preferably in exon 3 of SEQ ID NO: 12, leading to an alteration of the amino acid sequence of the CENH3 protein of *Brassica napus* after amino acid at position 37 of SEQ ID NO: 14, or a splicing error, preferably in intron 8 of SEQ ID NO: 12, leading to an alteration of the amino acid sequence of the CENH3 protein of *Brassica napus* after amino acid at position 163 of SEQ ID NO: 14, or the at least one mutation causes a splicing error, preferably in intron 4 of SEQ ID NO: 18, leading to an alteration of the amino acid sequence of the CENH3 protein of *Zea mays* after amino acid at position 89 of SEQ ID NO: 20, a splicing error, preferably in intron 5 of SEQ ID NO: 18, leading to an alteration of the amino acid sequence of the CENH3 protein of *Zea mays* after amino acid at position 115 of SEQ ID NO: 20, or a splicing error, preferably in intron 6 of SEQ ID NO: 18, leading to an alteration of the amino acid sequence of the CENH3 protein of *Zea mays* after amino acid at position 141 of SEQ ID NO: 20, or the at least one mutation causes a splicing error, preferably in intron 1 of SEQ ID NO: 15, leading to an alteration of the amino acid sequence of the CENH3 protein of *Sorghum bicolor* after amino acid at position 26 of SEQ ID NO: 17.

As further disclosed but not claimed, the at least one mutation causes an alteration of the amino acid sequence of the CENH3 protein and said alteration confers the biological activity of a haploid inducer, wherein the alteration is an insertion or deletion of one or more amino acids. The insertion can be introduced for instances by transposon mutagenesis and deletion can be created for instances by genomic engineering. Insertion and deletion can occur in any nucleotide sequence encoding one of the above described segments, in a nucleotide sequence of an intron or in a nucleotide sequence of the 5' untranslated region (UTR) or 3' UTR of the CENH3 gene, wherein the 5' UTR is located upstream from the nucleotide sequence encoding the N terminal tail domain and the 3' UTR is located downstream from the nucleotide sequence encoding the C-terminal domain. In any case the Insertion or deletion causes an alteration of the amino acid sequence of the CENH3 protein and said alteration confers the biological activity of a haploid inducer. The insertion can have a length of at least 1 nucleotide, at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 12 nucleotides, at least 14 nucleotides, at least 16 nucleotides, at least 18 nucleotides, at least 20 nucleotides, at least 25 nucleotides, at least 30 nucleotides, at least 40 nucleotides, at least 50 nucleotides, at least 75 nucleotides, at least 100 nucleotides, at least 200 nucleotides, at least 300 nucleotides, or at least 500 nucleotides.

In the context of the present invention the term 'at least one mutation' refers to preferably one mutation, in particular solely one mutation. In a further preferred embodiment, the term 'at least one mutation' refers to two mutations, in particular solely two mutations. In a further preferred embodiment, the term 'at least one mutation' refers to three mutations, in particular solely three mutations. In a further preferred embodiment, the term 'at least one mutation' refers to four mutations, in particular solely four mutations. In a further preferred embodiment, the term 'at least one mutation' refers to five mutations, in particular solely five mutations. In case of more than one mutation, mutations can occur also in different polynucleotides and causes alteration of the amino acid sequences of different CENH3 protein if existing for the specific plant species. For example, *Hordeum vulgare* have two different CENH3 proteins.

As further disclosed but not claimed, the at least one mutation is at least one mutation, is at least two mutations, is at least three mutations, is at least four mutations or is at least five mutations.

As further disclosed but not claimed, one segment of the CENH3 protein one amino acid substitution, in particular solely one amino acid substitution, is present.

As further disclosed but not claimed, one segment of the CENH3 protein two amino acid substitutions, in particular solely two amino acid substitutions, are present.

As further disclosed but not claimed, one segment of the CENH3 protein three amino acid substitutions, in particular solely three amino acid substitutions, are present.

As further disclosed but not claimed, one segment of the CENH3 protein four amino acid substitutions, in particular solely four amino acid substitutions, are present.

As further disclosed but not claimed, one segment of the CENH3 protein five amino acid substitutions, in particular solely five amino acid substitutions, are present.

As further disclosed but not claimed, in one segment of the CENH3 protein 1, 1 or 2, 1 to 3, 1 to 4, 1 to 5, preferably 1 to 6, and more preferably 1 to 7 amino acid substitutions are present.

In particular, the present disclosure is concerned partly with mutations that cause or lead to an amino acid substitution within a segment of the CENH3 protein. Thus, in this context a mutation is a non-synonymous point mutation or substitution in the DNA sequence encoding the CENH3 protein resulting in a change in amino acid. This is also called a missense mutation. Further, the change in amino acid or the amino acid substitution may be conservative, i.e. a change to an amino acid with similar physiochemical properties, semi-conservative, e.g. negative to positively charged amino acid, or radical, i.e. a change to a vastly different amino acid.

The disclosed plant having biological activity of a haploid inducer is homozygous with respect to the at least one mutation or heterozygous with respect to the at least one mutation.

The disclosed plant has the biological activity of a haploid inducer. This means that crossing between the plant and a wildtype plant or a plant expressing wildtype CENH3 protein yields at least 0.1 %, 0.2 %, 0.3 %, 0.4 %, 0.5 %, 0.6 %, 0.7 %, 0.8 %, 0.9 %, preferably at least 1 %, preferably at least 2 %, preferably at least 3 %, preferably at least 4 %, preferably at least 5 %, preferably at least 6 %, preferably at least 7 %, preferably at least 8 %, preferably at least 9 %, most preferred at least 10 %, at least 15 %, at least 20% or more haploid progeny. Thereby, a wildtype plant is preferably a plant of the same species which does not comprise the at least one mutation of the dislcosed plant within the corresponding endogenous CENH3 gene, i.e. the plant is able to express the native CENH3 protein, and a plant expressing wildtype CENH3 is preferably a plant of the same species which comprises i) a polynucleotide comprising a nucleotide sequence encoding the CENH3 protein without the at least one mutation of the plant according to the present invention and is able to express said native CENH3 protein or ii) a polynucleotide comprising a nucleotide sequence encoding a CENH3 protein from another plant species that shows a comparable functionality to the native CENH3, for instance, such CENH3 protein derived from another plant species can be introduced as a transgene.

Thus, the present disclosure most advantageously provides means and methods to generate haploid inducer lines in a wide range of eudicot, dicot and monocot species. The disclosure also allows the exchange of maternal cytoplasm and to create for instance cytoplasmic male sterility plants with a desired genotype in a single process step. The present invention is advantageous insofar as a single amino acid mutation can be generated by mutagenesis or any other non-GMO-based approaches.

Thus, the entire process of haploidization via application of a haploid inducer line characterized by a mutated endogenous CENH3 gene with an alteration of the amino acid at least one of the positions provided by the present invention may be non-transgenic.

In the context of the present invention, an "endogenous" gene, allele or protein refers to a non-recombinant sequence of a plant as the sequence occurs in the respective plant, in particular wildtype plant. The term "mutated" refers to a human-altered sequence. Examples of human-induced non-transgenic mutation include exposure of a plant to a high dose of chemical, radiological, or other mutagen for the purposes of selecting mutants. Alternatively, human-induced transgenic mutations, i.e. recombinant alterations or genomic engineering for example by means of TALE nucleases, zinc-finger nucleases or a CRISPR/Cas system, include fusions, insertions, deletions, and/or changes to the DNA or amino acid sequence.

A polynucleotide or polypeptide sequence is "heterologous or exogenous to" an organism if it originates from a foreign species, or, if from the same species, is modified from its original form. "Recombinant" refers to a human-altered, i.e. transgenic polynucleotide or polypeptide sequence. A "transgene" is used as the term is understood in the art and refers to a, preferably heterologous, nucleic acid introduced into a cell by human molecular manipulation of the cell's genome, e.g. by molecular transformation. Thus, a "transgenic plant" is a plant comprising a transgene, i.e. is a genetically-modified plant. The transgenic plant can be the initial plant into which the transgene was introduced as well as progeny thereof whose genome contains the transgene as well.

The term 'nucleotide sequence encoding' refers to a nucleic acid which directs the expression of a specific protein, in particular the CENH3 protein or parts thereof. The nucleotide sequences include both the DNA strand sequence that is transcribed into RNA and the RNA sequence that is translated into the protein. The nucleotide sequences include both the full-length nucleic acid sequences as well as non-full-length sequences derived from the full-length sequences.

The term 'gene' refers to a coding nucleotide sequence and associated regulatory nucleotide sequences, intron(s), 5' UTR and/or 3' UTR.

The term 'regulatory element' refers to a sequence, preferably a nucleotide sequence, located upstream (5'), within and/or downstream (3') to a nucleotide sequence, preferably a coding sequence, whose transcription and expression is controlled by the regulatory element, potentially in conjunction with the protein biosynthetic apparatus of the cell. 'Regulation' or 'regulate' refer to the modulation of the gene expression induced by DNA sequence elements located primarily, but not exclusively upstream (5') from the transcription start of the gene of interest. Regulation may result in an all-or-nothing response to a stimulation, or it may result in variations in the level of gene expression.

A regulatory element, in particular DNA sequence, such as a promoter is said to be "operably linked to" or "associated with" a DNA sequence that codes for a RNA or a protein, if the two sequences are situated and orientated such that the regulatory DNA sequence effects expression of the coding DNA sequence.

A 'promoter' is a DNA sequence initiating transcription of an associated DNA sequence, in particular being located upstream (5') from the start of transcription and being involved in recognition and being of the RNA-polymerase. Depending on the specific promoter region it may also include elements that act as regulators of gene expression such as activators, enhancers, and/or repressors.

A '3' regulatory element' (or '3' end') refers to that portion of a gene comprising a DNA segment, excluding the 5' sequence which drives the initiation of transcription and the structural portion of the gene, that determines the correct termination site and contains a polyadenylation signal and any other regulatory signals capable of effecting messenger RNA (mRNA) processing or gene expression. The polyadenylation signal is usually characterized by effecting the addition of polyadenylic acid tracts to the 3' end of the mRNA precursor. Polyadenylation signals are often recognized by the presence of homology to the canonical form 5'-AATAAA-3'.

The term 'coding sequence' refers to that portion of a gene encoding a protein, polypeptide, or a portion thereof, and excluding the regulatory sequences which drive the initiation or termination of transcription.

The gene, coding sequence or the regulatory element may be one normally found in the cell, in which case it is called 'autologous' or 'endogenous', or it may be one not normally found in a cellular location, in which case it is termed 'heterologous', 'transgenic' or 'transgene'.

A 'heterologous' gene, coding sequence or regulatory element may also be autologous to the cell but is, however, arranged in an order and/or orientation or in a genomic position or environment not normally found or occurring in the cell in which it is transferred.

The term 'vector' refers to a recombinant DNA construct which may be a plasmid, virus, autonomously replicating sequence, an artificial chromosome, such as the bacterial artificial chromosome BAC, phage or other nucleotide sequence, in which at least two nucleotide sequences, at least one of which is a nucleic acid molecule of the present invention, have been joined or recombined. A vector may be linear or circular. A vector may be composed of a single or double stranded DNA or RNA.

The term 'expression' refers to the transcription and/or translation of an endogenous gene or a transgene in plants.

'Transformation', 'transforming' and 'transferring' refers to methods to transfer nucleic acid molecules, in particular DNA, into cells including, but not limited to, biolistic approaches such as particle bombardment, microinjection, permeabilising the cell membrane with various physical, for instance electroporation, or chemical treatments, for instance polyethylene glycol or PEG, treatments; the fusion of protoplasts or Agrobacterium tumefaciens or rhizogenes mediated trans-formation. For the injection and electroporation of DNA in plant cells there are no specific requirements for the plasmids used. Plasmids such as pUC derivatives can be used. If whole plants are to be regenerated from such transformed cells, the use of a selectable marker is preferred. Depending upon the method for the introduction of desired genes into the plant cell, further DNA sequences may be necessary; if, for example, the Ti or Ri plasmid is used for the transformation of the plant cell, at least the right border, often, however, the right and left border of the Ti and Ri plasmid T-DNA have to be linked as flanking region to the genes to be introduced. Preferably, the transferred nucleic acid molecules are stably integrated in the genome or plastome of the recipient plant.

In the context of the present invention the term 'biological activity of a haploid inducer' or 'haploid inducer' or 'haploid inducer line' refers to a plant or plant line having the capability to produce haploid progeny or offspring in at least 0.1 %, at least 0.2 %, 0.3 %, 0.4 %, 0.5 %, 0.6 %, 0.7 %, 0.8 %, 0.9 %, preferably at least 1 %, preferably at least 2 %, preferably at least 3 %, preferably at least 4 %, preferably at least 5 %, preferably at least 6 %, preferably at least 7 %, preferably at least 8 %, preferably at least 9 %, most preferred at least 10 %, most preferred at least 15 %, most preferred at least 20 % of cases when crossed to a wildtype plant or a plant at least expressing wildtype CENH3 protein. Since the chromosomes of the haploid inducer are eliminated during meiosis the resulting haploid progeny only comprises the chromosomes of the wildtype parent. However, in case the haploid inducer was the ovule parent of the cross, the haploid progeny possesses the cytoplasm of the inducer and the chromosomes of the wildtype parent.

The term 'plant' according to the present invention includes whole plants or parts of such a whole plant.

Whole plants preferably are seed plants, or a crop. Parts of a plant are e.g. shoot vegetative organs/structures, e.g., leaves, stems and tubers; roots, flowers and floral organs/structures, e.g. bracts, sepals, petals, stamens, carpels, anthers and ovules; seed, including embryo, endosperm, and seed coat; fruit and the mature ovary; plant tissue, e.g. vascular tissue, ground tissue, and the like; and cells, e.g. guard cells, egg cells, trichomes and the like; and progeny of the same.

In any case, the plant of the present disclosure comprises at least one cell comprising a polynucleotide which comprises a nucleotide sequence encoding a centromer histone H3 (CENH3) protein, wherein the polynucleotide comprises at least one mutation causing an alteration of the amino acid sequence of the CENH3 protein and said alteration confers the biological activity of a haploid inducer, preferably as specified herein in more detail. Most preferably, most or in particular all cells of the plant of the present invention comprises the mutation(s) as described herein.

The species of plants that can be used in the method of the invention are preferably eudicot, dicot and monocot plants.

The term 'plant' in a preferred embodiment relates solely to a whole plant, i.e. a plant exhibiting the full phenotype of a developed plant and capable of reproduction, a developmental earlier stage thereof, e.g. a plant embryo, or to both.

In an embodiment of the present invention the term 'plant' refers to a part of a whole plant, in particular plant material, plant cells or plant cell cultures.

The term 'plant cell' describes the structural and physiological unit of the plant, and comprises a protoplast and a cell wall. The plant cell may be in form of an isolated single cell, such as a stomatal guard cell or a cultured cell, or as a part of a higher organized unit such as, for example, a plant tissue, or a plant organ.

The term 'plant material' includes plant parts, in particular plant cells, plant tissue, in particular plant propagation material, preferably leaves, stems, roots, emerged radicles, flowers or flower parts, petals, fruits, pollen, pollen tubes, anther filaments, ovules, embryo sacs, egg cells, ovaries, zygotes, embryos, zygotic embryos per se, somatic embryos, hypocotyl sections, apical meristems, vascular bundles, pericycles, seeds, roots, cuttings, cell or tissue cultures, or any other part or product of a plant.

Thus, the present disclosure also provides plant propagation material of the plants as disclosed. Said "plant propagation material" is understood to be any plant material that may be propagated sexually or asexually in vivo or in vitro. Particularly preferred within the scope of the disclosure are protoplasts, cells, calli, tissues, organs, seeds, embryos, pollen, egg cells, zygotes, together with any other propagating material obtained from transgenic plants. Parts of plants, such as for example flowers, stems, fruits, leaves, roots originating in mutated plants or their progeny previously mutated, preferably transformed, by means of the method of generating a plant of the present invention and therefore consisting at least in part of mutated cells, are also an object of the present invention.

Preferably, the plant according to the present disclosure is selected from the group consisting of barley (*Hordeum vulgare*), sorghum (*Sorghum bicolor*), rye (*Secale cereale*), Triticale, sugar cane (*Saccharum officinarium*), maize (*Zea mays*), foxtail millet (*Setaria italic*), rice (*Oryza sativa*), *Oryza minuta, Oryza australiensis, Oryza alta,* wheat (*Triticum aestivum*), *Triticum durum, Hordeum bulbosum,* purple false brome (*Brachypodium distachyon*), sea barley (*Hordeum marinum*)*,* goat grass (*Aegilops tauschii*)*,* apple (*Malus domestica*), *Beta vulgaris,* sunflower (*Helianthus annuus*), Australian carrot (*Daucus glochidiatus*), American wild carrot (*Daucus pusillus*), *Daucus muricatus,* carrot (*Daucus carota*), eucalyptus (*Eucalyptus grandis*)*, Erythranthe guttata, Genlisea aurea,* woodland tobacco (*Nicotiana sylvestris*), tobacco (*Nicotiana tabacum*), *Nicotiana tomentosiformis,* tomato (*Solanum lycopersicum*), potato (*Solanum tuberosum*), coffee (*Coffea canephora*), grape vine (*Vitis vinifera*), cucumber (*Cucumis sativus*), mulberry (*Morus notabilis*), thale cress (*Arabidopsis thaliana*), *Arabidopsis lyrata,* sand rock-cress (*Arabidopsis arenosa*), *Crucihimalaya himalaica, Crucihimalaya wallichii,* wavy bittercress (*Cardamine flexuosa*), peppergrass (*Lepidium virginicum*), sheperd's-purse (*Capsella bursa-pastoris*), *Olmarabidopsis pumila,* hairy rockcress (*Arabis hirsuta*), rape (*Brassica napus*), broccoli (*Brassica oleracea*), *Brassica rapa, Brassica juncacea,* black mustard (Brassica nigra), radish (Raphanus sativus), Eruca vesicaria sativa, orange (*Citrus sinensis*), *Jatropha curcas, Glycine max,* and black cottonwood (*Populus trichocarpa*).

Particularly preferred the plant is selected from the group consisting of barley (*Hordeum* v*ulgare*), sorghum (*Sorghum bicolor*), rye (*Secale cereale*), Triticale, sugar cane (*Saccharum officinarium*), maize (*Zea mays*), rice (*Oryza sativa*), wheat (*Triticum aestivum*), *Triticum durum, Avena sativa, Hordeum bulbosum, Beta vulgaris,* sunflower (*Helianthus annuus*), carrot (*Daucus carota*), tobacco (*Nicotiana tabacum*), tomato (*Solanum lycopersicum*), potato (*Solanum tuberosum*), coffee (*Coffea canephora*), grape vine (*Vitis vinifera*), cucumber (*Cucumis sativus*), thale cress (*Arabidopsis thaliana*), rape (*Brassica napus*), broccoli (*Brassica oleracea*), *Brassica rapa, Brassica juncacea,* black mustard (*Brassica nigra*), radish (Raphanus sativus), and *Glycine max.*

The plant according to the present disclosure may contain the polynucleotide which comprises the nucleotide sequence encoding the CENH3 either as an endogenous gene or a transgene.

The disclosure relates to a plant according to the present teaching, wherein the at least one amino acid substitution is introduced into the nucleotide sequence encoding CENH3 non-transgenically or transgenically.

Thus, in case the at least one mutation is effected in the endogenous CENH3 gene, the obtained plant is non-transgenic. The mutation may be effected via non-transgenic mutagenesis, transposon mutagenesis, in particular chemical mutagenesis, preferably via EMS (ethylmethane sulfonate)-induced TILLING or targeted genome editing.

As further disclosed but not claimed, the non-transgenic introduction of the at least one mutation causing an alteration of the amino acid sequence of the CENH3 protein and said alteration confers the biological activity of a haploid inducer may be effected via chemical mutagenesis, in particular via TILLING.

In another preferred embodiment, the at least one mutation is introduced into the plant in form of a transgene. Preferably, this is done by transforming a vector comprising a polynucleotide which comprises a nucleotide sequence encoding at least segment of CENH3 protein comprising at least one alteration of the amino acid sequence, preferably such as described herein. Methods for transformation of a plant and introducing a transgene into the genome of a plant are well-known in the prior art.

As further disclosed but not claimed, the transgenic introduction of the alteration into the amino acid sequence of the CENH3 protein is effected via transformation of a vector comprising polynucleotide which comprises a nucleotide sequence encoding at least segment of CENH3 protein or the CADT domain of the CENH3 protein comprising at least one alteration of the amino acid sequence, preferably comprising at least one amino acid substitution of one of the specified amino acids of consensus sequence SEQ ID NO: 1, 2, 3, 4, 5, 6, 7, 8 or 9, or as defined in Table 1, Table 2, Table 3, Table 4, Table 5, Table 6, Table 7 or Table 8.

Preferably, the Agrobacterium mediated transformation, floral dip method or particle bombardment are used for transformation.

In the preferred embodiment, wherein the polynucleotide comprising the nucleotide sequence encoding the altered CENH3 protein according to the present invention is transformed into the plant in form of a transgene and one or two alleles of the endogenous CENH3 gene are preferably inactivated or knocked out. Another preferred embodiment, wherein the polynucleotide comprising the nucleotide sequence encoding the altered amino acid sequence of CENH3 protein according to the present invention is transformed into the plant in form of a transgene and the transgene is overexpressed in order to be more competitive as the endogenous CENH3 protein and preferred during generation of a kinetochore complex.

The present invention also provides a plant obtainable, in particular obtained, by a method according to the present invention and which is characterized by having the biological activity of a haploid inducer.

In a preferred embodiment of the present invention, the method of producing the plant having biological activity of a haploid inducer according to the present invention is not an essentially biological method.

Further disclosed but not claimed, is a method of generating the plant having biological activity of a haploid inducer according to the disclosure, comprising the steps of:
i) subjecting seeds of a plant to a sufficient amount of the mutagen ethylmethane sulfonate (EMS) to obtain M1 plants,
ii) allowing sufficient production of fertile M2 plants,
iii) isolating genomic DNA of M2 plants and
iv) selecting individuals possessing at least mutation causing an alteration of the amino acid sequence of CENH3.

Further disclosed but not claimed, is a method of generating a plant having biological activity of a haploid inducer according to the disclosure, comprising the steps of:
xx) providing a vector comprising polynucleotide which comprises a nucleotide sequence encoding at least a segment of amino acid sequence of a CENH3 protein, wherein the polynucleotide comprises at least one mutation causing an alteration of the amino acid sequence of the CENH3 protein,
yy) transforming a plant cell with the vector, wherein preferably the plant cell comprising one or two endogenous alleles of a CENH3 gene inactivated or knocked out, and
zz) regenerating a plant having the biological activity of a haploid inducer from the plant cell.

Further disclosed but not claimed, is a method of generating a plant having biological activity of a haploid inducer according to the disclosure, comprising the steps of:
yy) transforming a plant cell with a polynucleotide which comprises a nucleotide sequence encoding at least a segment of amino acid sequence of a CENH3 protein, wherein the polynucleotide comprises at least one mutation causing an alteration of the amino acid sequence of the CENH3 protein or a vector comprising polynucleotide which comprises a nucleotide sequence encoding at least a segment of amino acid sequence of a CENH3 protein, wherein the polynucleotide comprises at least one mutation causing an alteration of the amino acid sequence of the CENH3 protein, and
zz) regenerating a plant having the biological activity of a haploid inducer from the plant cell.

In particular, the disclosure relates to a haploid plant, obtainable, in particular obtained, by:
a) a cross of a plant having the biological activity of a haploid inducer according to the disclosure with a plant expressing wildtype CENH3 protein, and optionally
b) identifying haploid progeny generated from the crossing step.

Preferably, the identified haploid plant may be converted into a double haploid plant, preferably via colchicine treatment, which is also part of the disclosure. Thus, the disclosure also relates to a double-haploid plant, obtainable, in particular obtained, by converting the haploid plant according to the disclosure into a double haploid plant, preferably via colchicine treatment or via spontaneous chromosome doubling.

Further disclosed but not claimed, is a method of generating a haploid plant, comprising the steps of:
a) crossing a plant having the biological activity of a haploid inducer according to the disclosure to a plant expressing wildtype CENH3 protein and
b) identifying haploid progeny generated from the crossing step.

In a further step c) the selected haploid plant may be converted into a double haploid plant, preferably via colchicine treatment. Thus, the disclosure relates also to a method of generating a double haploid plant.

In a disclosure, the method provided is not an essentially biological method.

In particular, the present methods do not rely solely on, in particular do not consist of, natural phenomena such as crossing or selection, but in fact are essentially based on the technical teaching so as to provide a specifically mutated nucleotide sequence prepared by mankind's contribution. Thus, the present invention introduces a specific structural feature, namely a mutation, into a nucleotide sequence and a plant of the present invention, which mutation is not caused by or associated with any natural phenomena such as crossing or selection.

Further disclosed but not claimed, is a method including a crossing step, said crossing step does not provide - such as a crossing usually does - heterozygous progeny but in fact homozygous progeny. Furthermore, the haploidy of progeny is not the result of the mixing of genes of the plants used for sexual crossing. Furthermore, the presently disclosed process of generating a double haploid plant cannot be found in nature.

Further disclosed but not claimed, is a method of facilitating a cytoplasm exchange, comprising the steps of:
x) crossing a plant according to the disclosure as ovule parent to a plant expressing wildtype CENH3 protein as pollen parent, and
y) obtaining a haploid progeny plant comprising the chromosomes of the pollen parent and the cytoplasm of ovule parent.

Preferably, the method provided is not an essentially biological method. Said method is not a biological method essentially for the same reasons as indicated above, in particular since it is not entirely made up of natural phenomena such as crossing and selection but involves as an essential feature a significant technical teaching so as to provide a particular mutation in a nucleotide sequence and a plant of the present invention. Furthermore, the haploidy of the progeny is not the result of the mixing of genes of the plants used for sexual crossing.

The method can advantageously be used to create cytoplasmic male sterility (CMS). CMS is caused by the extranuclear genome (mitochondria or chloroplasts) and shows maternal inheritance. Thus, the plant according to the disclosure has to exhibit CMS and be the ovule parent of the cross. In this way CMS can be introduced into the crossing partner, preferably being an elite line of a crop.

The plant according to the disclosure may also be used in a method to restore male fertility by providing a normal cytoplasm to a crossing partner that is CMS. Through such a cross the chromosomes of the CMS plant are introduced into the normal cytoplasm of the haploid inducer of the disclosure which is not CMS. However, pollen production of the CMS plant has to be induced via temperature, light, length of day etc.

Without being bound by theory a possible model of how the present methods, in particular a method of uniparental chromosome elimination, works in *inducer CENH3* x *wild type CENH3* interspecific hybrid embryos could work as follows: (A) Likely haploid inducer-derived egg cells contain either less CENH3 or compared to wild type a reduced unknown 'CENH3-transgeneration required signature'. A reduced amount of maternal CENH3 is less likely as according to studies performed with a CENH3-GFP reporter in *A. thaliana* plants sperm nuclei but not eggs cells are marked by CENH3. However, it is still possible that residual maternal CENH3s, generating a 'centromeric imprinting' are transmitted to the progeny. (B) Within a few hours after fertilization also paternal wild type CENH3 is actively removed from the zygote nucleus, and (C) centromeric reloading of CENH3-GFP in the zygote occurs at the 16-nuclei stage of endosperm development in *A. thaliana.* (D) In embryos undergoing haploidization centromeric reloading of the maternal chromosomes is impaired or delayed causing lagging chromosomes because of centromere inactivity during anaphase. Subsequently micronucleated *haploid inducer* chromosomes will degrade and (E) a haploid embryo will develop. Haploid embryos contain paternal-derived chromosomes in the background of maternal-derived cytoplasm.

The present invention also relates to a polynucleotide which comprises a nucleotide sequence encoding a CENH3 protein, wherein the polynucleotide comprises at least one mutation causing an alteration of the amino acid sequence of the CENH3 protein, and said alteration confers the biological activity of a haploid inducer, wherein the at least one mutation causes a substitution of a specified amino acid in the loop2, wherein
i. the amino acid arginine at position 159 of SEQ ID NO: 14 may be substituted for histidine, or
ii. the amino acid valine at position 160 of SEQ ID NO: 14 may be substituted for isoleucine, or
iii. the amino acid threonine at position 139 of SEQ ID NO: 20 may be substituted for isoleucine.

The present invention also relates to a vector, in particular viral vector, construct or plasmid comprising said polynucleotide and, if present, associated sequences, preferably as indicated herein.

In a particularly preferred embodiment of the present invention, the polynucleotide which comprises the nucleotide sequence encoding a segment of the CENH3 protein preferably comprises at least the complete coding region of CENH3, in particular the gene of CENH3.

In a furthermore preferred embodiment of the present invention, polynucleotide or the coding sequence of the CENH3 may be associated with regulatory elements, such as 5'-and/or 3'- regulatory elements, most preferably with a promoter, preferably a constitutive or inducible promoter.

Further, a plant cell comprising said polynucleotide or a vector comprising it as a transgene is provided by the present invention.

In the context of the present invention, the term 'comprising' as used herein is understood as to have the meaning of 'including' or 'containing', which means that in addition to the explicitly mentioned element further elements are possibly present.

In a preferred embodiment of the present invention, the term 'comprising' as used herein is also understood to mean 'consisting of' thereby excluding the presence of other elements besides the explicitly mentioned element.

In a furthermore preferred embodiment, the term 'comprising' as used herein is also understood to mean 'consisting essentially of' thereby excluding the presence of other elements providing a significant contribution to the disclosed teaching besides the explicitly mentioned element.

The sequence protocol shows:
SEQ ID NO: 1: the amino acid consensus sequence of the N-terminal tail domain of the CENH3 (part A),
SEQ ID NO: 2: the amino acid consensus sequence of the N-terminal tail domain of the CENH3 (part B),
SEQ ID NO: 3: the amino acid consensus sequence of the aN-helix of the CENH3,
SEQ ID NO: 4: the amino acid consensus sequence of the α1-helix of the CENH3,
SEQ ID NO: 5: the amino acid consensus sequence of the loop1 of the CENH3,
SEQ ID NO: 6: the amino acid consensus sequence of the α2-helix of the CENH3,
SEQ ID NO: 7: the amino acid consensus sequence of the loop2 of the CENH3,
SEQ ID NO: 8: the amino acid consensus sequence of the α3-helix of the CENH3,
SEQ ID NO: 9: the amino acid consensus sequence of the C-terminal domain of the CENH3,
SEQ ID NO: 10: the nucleotide sequence of the wildtype coding sequence (cDNA) of *A. thaliana* CENH3,
SEQ ID NO: 11: the amino acid sequence of the wildtype *A. thaliana* CENH3,
SEQ ID NO: 12: the nucleotide sequence of the wildtype genomic sequence (genomic DNA) of B. *napus* CENH3,
SEQ ID NO: 13: the nucleotide sequence of the wildtype coding sequence (cDNA) of B. *napus* CENH3,
SEQ ID NO: 14: the amino acid sequence of the wildtype B. *napus* CENH3,
SEQ ID NO: 15: the nucleotide sequence of the wildtype genomic sequence (genomic DNA) of *S. bicolor* CENH3,
SEQ ID NO: 16: the nucleotide sequence of the wildtype coding sequence (cDNA) of S. *bicolor* CENH3,
SEQ ID NO: 17: the amino acid sequence of the wildtype *S. bicolor* CENH3,
SEQ ID NO: 18: the nucleotide sequence of the wildtype genomic sequence (genomic DNA) of Z. *mays* CENH3,
SEQ ID NO: 19: the nucleotide sequence of the wildtype coding sequence (cDNA) of Z. *mays* CENH3,
SEQ ID NO: 20: the amino acid sequence of the wildtype Z. *mays* CENH3,
SEQ ID NO: 21: the nucleotide sequence of the wildtype genomic sequence (genomic DNA) of B. *vulgaris* CENH3,
SEQ ID NO: 22: the nucleotide sequence of the wildtype coding sequence (cDNA) of B. *vulgaris* CENH3,
SEQ ID NO: 23: the amino acid sequence of the wildtype B. *vulgaris* CENH3, and
SEQ ID NO: 24: the nucleotide sequence of the genomic sequence (genomic DNA) of Z. *mays* CENH3-Mu-mutation.

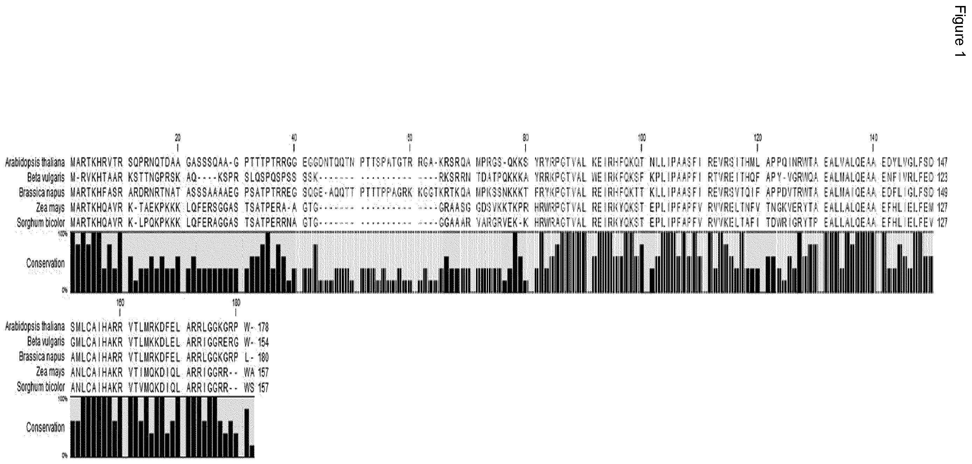

The Figure shows an alignment of the amino acid sequences of *Arabidopsis thaliana* (first row), *Beta vulgaris* (second row), *Brassica napus* (third row), *Zea mays* (forth row), *Sorghum bicolor* (fifth row) as well as a diagram showing the level of conservation over these five plant species.

### Examples

### Identification of CENH3 mutants

For the identification of mutations within the gene of CENH3 which cause an alteration of the amino acid sequence of the translated CENH3, wherein the alteration is able to confer the biological activity of a haploid inducer to a plant, all segments of the CENH3 gene has been investigated with respect to suitable mutations, even if Ravi und Chan 2010 highlighted only the particular importance of the N terminal domain. First own investigation on mutants in other segments like α2-helix (not yet published) gave indications that in addition the modification of other segments can result in a destabilization of the CENH3 binding capacities to DNA.

In order to find mutant CENH3 genes in different plants species Tilling populations having high mutation rates have generated for corn (*Zea mays*), rape seed (*Brassica napus*), sorghum (*Sorghum bicolor*) and sugar beet (*Beta vulgaris*) and have been screened for CENH3 mutations. For that, after development of amplicons covering all exons of the CENH3 genes 1000-10000 plants per plant species have been analyzed by means of Sanger's sequencing method. In addition, M2 sugar beet plants have been tested for mutations using specific PCR.

Furthermore, the affect of the identified mutation within the CENH3 gene on the primary and secondary structure of the encoded protein have been evaluated using inter alia the software Prof (Rost, B. and Sander, C. (1994a). Combining evolutionary information and neural networks to predict protein secondary structure. Proteins, 19(1), 55-72. Rost, B. and Sander, C. (1994b). Conservation and prediction of solvent accessibility in protein families. Proteins, 20(3), 216-26. Rost, B., Casadio, R., Fariselli, P., and Sander, C. (1995). Transmembrane helices predicted at 95% accuracy. Protein Sci, 4(3), 521-33.). Tables 9 to 12 show the identified mutations in *B. napus, Z. mays, S. bicolor* and B. *vulgaris,* respectively, which are separated in mutations causing a splicing error and in mutations causing an amino acid substitution. A mutation within a splicing site is of particular interest. Such mutation(s) can cause a malfunctioning splicing site (splicing error), which then results in an increased cellular translational production of non-fully functional CENH3 protein, which shows e.g. an impaired stability, a reduced binding affinity to DNA, a changed geometric shape of the protein, preferably a changed secondary or tertiary structure, or a disordered protein folding compared to the fully functional wildtype CENH3 protein. Plants having a genome which was heterozygous for such mutation(s) were viable.

**Table 9: mutation of the CENH3 derived from Brassica napus (aa: amino acid; nd: not determined, y: yes, n: no). Amino acid substitution is given as X#Y, i.e. amino acid X (one letter code) is substituted for amino acid Y at position #.**

| mutation identifier (Brassica napus) | codon wildtype | codon mutant | mutation | chance in secondary structure |
|---|---|---|---|---|
| BN_CenH3_01 | | | splicing error after aa at position18 | nd |
| BN_CenH3_02 | | | splicing error after aa at position 33 | nd |
| BN_CenH3_03 | | | splicing error after aa at position 37 | nd |
| BN_CenH3_04 | | | splicing error after aa at position 37 | nd |
| BN_CenH3_05 | | | splicing error after aa at position 163 | nd |
| BN_CenH3_06 | tcc | ttc | S9F | y |
| BN_CenH3_07 | cga | caa | R16Q | y |
| BN_CenH3_08 | tcg | ttg | S24L | y |
| BN_CenH3_09 | gaa | aaa | E29K | n |
| BN_CenH3_10 | ggt | gat | G30D | n |
| BN_CenH3_11 | gcg | acg | A33T | n |
| BN_CenH3_12 | ccg | ctg | P35L | y |
| BN_CenH3_13 | agc | aac | S41N | n |
| BN_CenH3_14 | gga | gaa | G43E | y |
| BN_CenH3_15 | cct | tct | P50S | n |
| BN_CenH3_16 | cca | cta | P55L | n |
| BN_CenH3_17 | ggt | gat | G57D | n |
| BN_CenH3_18 | gga | gaa | G61E | y |
| BN_CenH3_19 | cga | caa | R65Q | y |
| BN_CenH3_20 | cga | tga | R65stop | n |
| BN_CenH3_21 | cct | tct | P71S | y |
| BN_CenH3_22 | gcc | acc | A105T | y |
| BN_CenH3_23 | cga | caa | R110Q | y |
| BN_CenH3_25 | aqt | aat | S114N | y |
| BN_CenH3_26 | cct | tct | P121S | n |
| BN_CenH3_27 | tgg | tga | W127stop | n |
| BN_CenH3_28 | ctt | ttt | L132F | y |
| BN_CenH3_29 | gcg | acg | A138T | n |
| BN_CenH3_30 | tgc | tac | C153Y | y |
| BN_CenH3_31 | gct | gtt | A154V | y |
| BN_CenH3_32 | cgt | cat | R159H | n |
| BN_CenH3_33 | gtt | att | V160I | n |
| BN_CenH3_34 | gat | aat | D166N | n |
| BN_CenH3_35 | gag | aag | E168K | n |
| BN_CenH3_36 | cgt | cat | R172H | n |
| BN_CenH3_37 | ctt | ttt | L173F | n |
| BN_CenH3_38 | gga | gaa | G174E | y |
| BN_CenH3_39 | aga | aaa | R178K | n |

**Table 10: mutation of the CENH3 derived from Zea mays (aa: amino acid; nd: not determined, y: yes, n: no). Amino acid substitution is given as X#Y, i.e. amino acid X (one letter code) is substituted for amino acid Y at position #.**

| mutation identifier (Zea mays) | codon wildtype | codon mutant | mutation | chance in secondary structure |
|---|---|---|---|---|
| ZM_CenH3_01 | | | splicing error after aa at position 89 | nd |
| ZM_CenH3_02 | | | splicing error after aa at position 115 | nd |
| ZM_CenH3_03 | | | splicing error after aa at position 141 | nd |
| ZM_CenH3_04 | gcg | acg | A32T | nd |
| ZM_CenH3_05 | gaa | aaa | E35K | nd |
| ZM_CenH3_06 | cca | tca | P56S | nd |
| ZM_CenH3_07 | gca | aca | A107T | nd |
| ZM_CenH3_08 | caa | taa | Q114stop | nd |
| ZM_CenH3_09 | gga | gaa | G152E | nd |
| ZM_CenH3_10 | cgt | cat | R155H | nd |
| ZM_CenH3_11 | gtg | atg | V89M | nd |
| ZM_CenH3_12 | aca | ata | T139I | nd |

**Table 11: mutation of the CENH3 derived from Sorghum bicolor (aa: amino acid; nd: not determined, y: yes, n: no). Amino acid substitution is given as X#Y, i.e. amino acid X (one letter code) is substituted for amino acid Y at position #.**

| mutation identifier (S. bicolor) | codon wildtype | codon mutant | mutation | chance in secondary structure |
|---|---|---|---|---|
| SB_CenH3_01 | | | splicing error after aa at position 26 | nd |
| SB_CenH3_02 | gca | gta | A62V | nd |
| SB_CenH3_03 | act | agt | T64S | nd |
| SB_CenH3_04 | gca | gta | A95V | nd |
| SB_CenH3_05 | gca | aca | A25T | nd |
| SB_CenH3_06 | tcg | ttg | S157L | nd |

**Table 12: mutation of the CENH3 derived from Beta vulgaris (nd: not determined, y: yes, n: no). Amino acid substitution is given as X#Y, i.e. amino acid X (one letter code) is substituted for amino acid Y at position #.**

| mutation identifier (Beta vulgaris) | codon wildtype | codon mutant | mutation | chance in secondary structure |
|---|---|---|---|---|
| Bv_CENH3_01 | gat | aat | D46N | nd |
| Bv_CENH3_02 | gat | ggt | D46G | nd |
| Bv_CENH3_03 | aga | aaa | A2K | nd |
| Bv_CENH3_04 | ctg | cag | L106Q | nd |
| Bv_CENH3_05 | ctt | cct | L109P | nd |
| Bv_CENH3_06 | caa | cta | Q110L | nd |

Beside mutations of splicing sites and point mutations causing amino acid substitutions within the amino acid sequence of CENH3 protein a corn mutant (called Mu-mutant) has been identified that contains a transposon insertion within the 5' untranslated region of the CENH3 gene (see SEQ ID NO: 24). This mutation causes an extension of the N terminal tail domain. Thus, the effect of this mutation on CENH3 is very similar to the mutation described by Ravi & Chan (2010) except that the mutation is non-transgenic.

### Testing of CENH3 mutants

To evaluate the biological activity of a haploid inducer in the identified mutants and to test the maternal and paternal performance of haploid induction the mutant plants have to be crossed with another tester plant of the same species (carrying wildtype form of CENH3) that can be used as ovule parent or pollen parent, respectively. Putative haploid progeny from this cross can be determined quickly if the used tester lines carry a recessive non-CENH3 mutation. So, the haploid plants show the recessive phenotype. For example, in corn the manifestation of the mutation *glossy* (Mutants of maize, Neuffer, MG et al. 1997.Cold Spring Harbor Laboratory, New York) can be used.

Cytogenetic analyses of mitose and meiose with the inductors give indications for suitability of mutants as haploid inducers. The homozygosity is determined by use of molecular markers, polymorph for tester and potential inductor. Haploidy as such is tested cytogenetically.

In crossings with the tester plants the TILLING plants with mutated endogenous CENH3 gene as described above yield at least 0.4 % haploid progeny. Frequently but not always, the induction rate was higher if the tester was used as female parent in the cross.

For example, in *Brassica napus* the mutations that are based on amino acid substitutions in the N-terminal tail domain result in induction rates of at least 0.5% and partly up to more than 2%. Thereby, the locations of mutations are not specific to a certain region in this domain but rather distributed over the entire domain. The N-terminal tail domain in *Brassica napus* reaches from amino acid position 1 to 84. Mutations conferring the biological activity of a haploid inducer can be found for instances in positions 9, 16, 24, 29, 30, 33, 41, 43, 50, 55, 57 and 61, whereby not all of these mutations lead necessarily to a chance in secondary structure of the protein (calculated in silico). Comparable results have been achieved for the more conserved histone fold domain containing the three helices and the two loops. Even though over the entire histone fold domain suitable mutations can be found specifically amino acid substitutions in the α2-helix, the CATD domain and the loop2 yielded on average significantly higher induction rates. Due to these observations on the N-terminal tail domain and the histone fold domain, it can be assumed that also other not tested positions and other not tested amino substitutions will confer the same or even an improved haploid inductivity. Further, another kind of modification of the endogenous CENH3 gene is the substitution of nucleotides in splicing sites what consequently leads to splicing errors. Such mutations are also suitable to confer the biological activity of a haploid inducer. The observed induction rates showed at least 0.5 % haploid progeny. Even here it can be assumed that also other not tested splicing sites will confer the same or even an improved haploid inductivity.

For example, in *Zea mays* the mutations that are based on amino acid substitutions in the N-terminal tail domain result in induction rates of at least 0.4%. Thereby, the locations of mutations are not specific to a certain region in this domain but rather distributed over the entire domain. The N-terminal tail domain in *Zea mays* reaches from amino acid position 1 to 62. Mutations conferring the biological activity of a haploid inducer can be found for instances in positions 32, 35 and 56. Comparable results have been achieved for the more conserved histone fold domain containing the three helices and the two loops. Due to these observations on the N-terminal tail domain and the histone fold domain, it can be assumed that also other not tested positions and other not tested amino substitutions will confer the same or even an improved haploid inductivity. Further, another kind of modification of the endogenous CENH3 gene is the substitution of nucleotides in splicing sites what consequently leads to splicing errors. Such mutations are also suitable to confer the biological activity of a haploid inducer. The observed induction rates showed at least 0.4 % haploid progeny. Even here it can be assumed that also other not tested splicing sites will confer the same or even an improved haploid inductivity.

In addition, the Mu-mutant containing a transposon insertion within the 5' untranslated region of the CENH3 gene (SEQ ID NO: 24) has been tested for biological activity of a haploid inducer. This non-transgenic mutation causes an induction rate of more than 1.0 %.

Moreover, the results of crossing across different crops demonstrate that identified and indicated mutations could be functional even in further plant species. Therefore mutations could be introduced into other plant species by techniques like TILLING, Mutagenesis or genome editing (e.g. CRISPR/Cas, TALENs, Zinc Finger nucleases *etc*.). Moreover, the biological activity and efficiency of a haploid inducer could be further improved by combining different identified mutations in one plant and/or modifying the genetic background of the haploid inducer. The combination of different mutations could be achieved efficiently by genome editing, or the mutant haploid inducer is mutagenized for a second time.

## Claims

1. An isolated polynucleotide comprising a nucleotide sequence encoding a centromer histone H3 (CENH3) protein, wherein the polynucleotide comprises at least one mutation causing an alteration of the amino acid sequence of the CENH3 protein and said alteration confers the biological activity of a haploid inducer, wherein the at least one mutation causes a substitution of a specified amino acid in the loop2, wherein
i. the amino acid arginine at position 159 of SEQ ID NO: 14 is substituted for histidine, or
ii. the amino acid valine at position 160 of SEQ ID NO: 14 is substituted for isoleucine, or
iii. the amino acid threonine at position 139 of SEQ ID NO: 20 is substituted for isoleucine.

2. A vector comprising the polynucleotide of claim 1.

3. A plant cell or host cell comprising the polynucleotide of claim 1 as a transgene or the vector of claim 2 as a transgene.

4. A method of generating a plant having biological activity of a haploid inducer, comprising the steps of:
a) transforming a plant cell with the polynucleotide of claim 1 or the vector of claim 2, and
b) regenerating a plant having the biological activity of a haploid inducer from the plant cell.

## Patentansprüche

1. Isoliertes Polynukleotid, umfassend eine Nukleotidsequenz, die für ein Centromer-His-ton-H3(CENH3)-Protein kodiert, wobei das Polynukleotid mindestens eine Mutation umfasst, die eine Veränderung der Aminosäuresequenz des CENH3-Proteins verursacht, und wobei die Veränderung die biologische Aktivität eines Haploidinduktors verleiht, wobei die mindestens eine Mutation eine Substitution einer spezifizierten Aminosäure in loop2 verursacht, wobei
i. die Aminosäure Arginin an Position 159 der SEQ ID NO: 14 durch Histidin substituiert ist, oder
ii. die Aminosäure Valin an Position 160 der SEQ ID NO: 14 durch Isoleucin substituiert ist, oder
iii. die Aminosäure Threonin an Position 139 der SEQ ID NO: 20 durch Isoleucin substituiert ist.

2. Vektor, umfassend das Polynukleotid nach Anspruch 1.

3. Pflanzenzelle oder Wirtszelle, umfassend das Polynukleotid nach Anspruch 1 als Transgen oder den Vektor nach Anspruch 2 als Transgen.

4. Verfahren zur Erzeugung einer Pflanze mit der biologischen Aktivität eines Haploidinduktors, umfassend die Schritte:
a) Transformieren einer Pflanzenzelle mit dem Polynukleotid nach Anspruch 1 oder dem Vektor nach Anspruch 2, und
b) Regenerieren einer Pflanze mit der biologischen Aktivität eines Haploidinduktors aus der Pflanzenzelle.

## Revendications

1. Polynucléotide isolé comprenant une séquence nucléotidique codant pour une protéine histone H3 centromérique (CENH3), dans lequel le polynucléotide comprend au moins une mutation provoquant une altération de la séquence d'acides aminés de la protéine CENH3, ladite altération conférant l'activité biologique d'un inducteur d'haploïdes, dans lequel ladite au moins une mutation provoque une substitution d'un acide aminé spécifié dans la boucle2 (loop2), dans lequel
i. l'acide aminé arginine en position 159 de SEQ ID NO : 14 est substitué par de l'histidine, ou
ii. l'acide aminé valine en position 160 de SEQ ID NO : 14 est substitué par de l'isoleucine, ou
iii. l'acide aminé thréonine en position 139 de SEQ ID NO : 20 est substitué par de l'isoleucine.

2. Vecteur comprenant le polynucléotide selon la revendication 1.

3. Cellule végétale ou cellule hôte comprenant le polynucléotide selon la revendication 1 en tant que transgène, ou le vecteur selon la revendication 2 en tant que transgène.

4. Procédé d'obtention d'une plante ayant l'activité biologique d'un inducteur d'haploïdes, comprenant les étapes consistant à :
a) transformer une cellule végétale avec le polynucléotide selon la revendication 1 ou le vecteur selon la revendication 2, et
b) régénérer une plante ayant l'activité biologique d'un inducteur haploïde à partir de la cellule végétale.
